# EUROPEAN PATENT SPECIFICATION

(11) **EP 3 326 218 B1**
(45) Date of publication and mention of the grant of the patent: **17.02.2021**
(21) Application number: 16734557.8
(22) Date of filing: 28.06.2016
(51) Int. Cl.: H01L 51/00, C07C 211/61

(54) **MATERIALS FOR ORGANIC ELECTROLUMINESCENT DEVICES**
MATERIALIEN FÜR ORGANISCHE ELEKTROLUMINESZENZVORRICHTUNGEN
MATIÈRES POUR DISPOSITIFS ÉLECTROLUMINESCENTS ORGANIQUES

(30) Priority: 22.07.2015 EP 15177867
(43) Date of publication of application: 30.05.2018
(73) Proprietor: Merck Patent GmbH, 64293 Darmstadt (DE)
(72) Inventor: MUJICA-FERNAUD, Teresa, 64283 Darmstadt (DE); MONTENEGRO, Elvira, 69469 Weinheim (DE); GROSSMANN, Tobias, 64297 Darmstadt (DE); PFISTER, Jochen, 64342 Seeheim-Jugenheim (DE)
(86) International application number: PCT/EP2016/001099
(87) International publication number: WO 2017/012687

(56) References cited:
- EP-A1- 3 010 066
- WO-A1-2012/034627
- WO-A1-2015/131976

## Description

The present invention relates to materials for use in electronic devices, in particular in organic electroluminescent devices, and to electronic devices comprising these materials.

The structure of organic electroluminescent devices (OLEDs) in which organic semiconductors are employed as functional materials is described, for example, in US 4,539,507, US 5,151,629, EP 0676461 and WO 98/27136. The emitting materials employed here are increasingly organometallic complexes which exhibit phosphorescence instead of fluorescence (M. A. Baldo et al., Appl. Phys. Lett. 1999, 75, 4-6).

In accordance with the prior art, the hole-transport materials used in the hole-transport layer or in the hole-injection layer are, in particular, triarylamine derivatives which frequently contain at least two triarylamino groups or at least one triarylamino group and at least one carbazole group. These compounds are frequently derived from diarylamino-substituted triphenylamines (TPA type), from diarylamino-substituted biphenyl derivatives (TAD type) or combinations of these base compounds. Furthermore, for example, use is made of spirobifluorene derivatives which are substituted by one to four diarylamino groups (for example in accordance with EP 676461, US 7,714,145, WO 2012/034627 A1 or EP2814906). In the case of these compounds, there is furthermore a need for improvement both in the case of fluorescent and in the case of phosphorescent OLEDs, in particular with respect to efficiency, lifetime and operating voltage on use in an organic electroluminescent device.

The object of the present invention is to provide compounds which are suitable for use in a fluorescent or phosphorescent OLED, in particular a phosphorescent OLED, for example as hole-transport material in a hole-transport or hole-injection or exciton-blocking layer or electron-blocking layer, or is comprised as matrix material for fluorescent or phosphorescent emitters in an emitting layer.

It has now been found that certain compounds described below in greater detail achieve this object and result in significant improvements in the organic electroluminescent device, in particular with respect to the lifetime, the efficiency and the operating voltage. This applies to phosphorescent and fluorescent electroluminescent devices, especially on use of the compounds according to the invention as hole-transport material, hole-injection material or as matrix material. Furthermore, the compounds described below exhibit a high thermal stability and can therefore be sublimed without decomposition and without a residue. In addition, they have a low crystallinity and a high glass transition temperature, which leads to an improved lifetime.

The invention is defined by the claims. Any subject-matter falling outside the scope of the claims is provided for information purposes only.

The present invention therefore relates to a compound of the following formula (1): 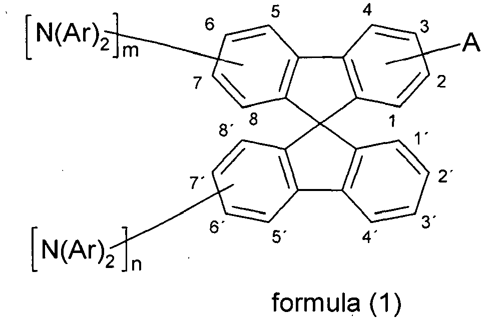 where A is a group of the following formula (2), which can only be present at the position 1 or position 4 of the spirobifluorene structure as depicted in formula (1) 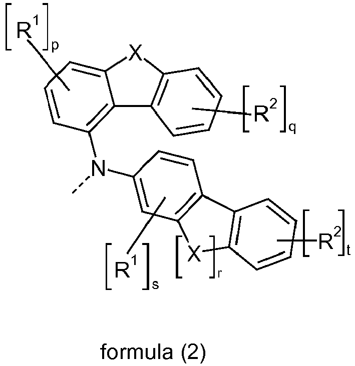
where the dashed bond indicates the bonding to the spirobifluorene structure;
where each free position 1 to 8 and 1' to 8' of the spirobifluorene structure, which is not substituted by a group A or N(Ar)₂, may be substituted by a group R;
and where the following applies to the symbols and indices used:
   X is a divalent bridge selected from the group consisting of C(R⁰)₂, Si(R⁰)₂, C=O, O, S, S=O and SO₂;
      or X is a group of the following formula (3), 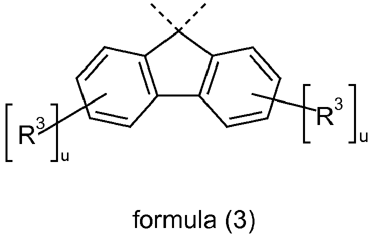 where the dashed bonds indicate the bonding to the fluorene group;
   R, R⁰ are selected on each occurrence, identically or differently, from the group consisting of H, D, F, Cl, Br, I, CHO, CN, C(=O)Ar¹, P(=O)(Ar¹)₂, S(=O)Ar¹, S(=O)₂Ar¹ NO², Si(R⁴)₃, B(OR⁴)₂, OSO₂R⁴, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁴, where in each case one or more non-adjacent CH₂ groups may be replaced by R⁴C=CR⁴, C=C, Si(R⁴)₂, Ge(R⁴)₂, Sn(R⁴)₂, C=O, C=S, C=Se, P(=O)(R⁴), SO, SO₂, O, S or CONR⁴ and where one or more H atoms may be replaced by D, F, Cl, Br, I, CN or NO², an aromatic or heteroaromatic ring system having 5 to 40 aromatic ring atoms, which may in each case be substituted by one or more radicals R⁴, an aryloxy or heteroaryloxy group having 5 to 40 aromatic ring atoms, which may be substituted by one or more radicals R⁴, a thioaryl or thio-heteroaryl group having 5 to 40 aromatic ring atoms, which may be substituted by one or more radicals R⁴, where two substituents R⁰ attached to the same C or Si atom may optionally form a mono- or polycyclic aliphatic ring system, which may be substituted by one or more radicals R⁴;
      R¹, R² and R³ are selected on each occurrence, identically or differently, from the group consisting of H, D, F, CI, Br, I, CHO, CN, N(Ar¹)₂, C(=O)Ar¹, P(=O)(Ar¹)₂, S(=O)Ar¹, S(=O)₂Ar¹, NO², Si(R⁴)₃, B(OR⁴)₂, OSO₂R⁴, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁴, where in each case one or more non-adjacent CH₂ groups may be replaced by R⁴C=CR⁴, C≡C, Si(R⁴)₂, Ge(R⁴)₂, Sn(R⁴)₂, C=O, C=S, C=Se, P(=O)(R⁴), SO, SO₂, O, S or CONR⁴ and where one or more H atoms may be replaced by D, F, Cl, Br, I, CN or NO₂, an aromatic or heteroaromatic ring system having 5 to 40 aromatic ring atoms, which may in each case be substituted by one or more radicals R⁴, an aryloxy or heteroaryloxy group having 5 to 40 aromatic ring atoms, which may be substituted by one or more radicals R⁴, a thioaryl or thio-heteroaryl group having 5 to 40 aromatic ring atoms, which may be substituted by one or more radicals R⁴, where two or more adjacent substituents R¹, two or more adjacent substituents R² or two or more adjacent substituents R³, may optionally form a mono- or polycyclic, aliphatic ring system or aromatic ring system, which may be substituted by one or more radicals R⁴;
   R⁴ is selected on each occurrence, identically or differently, from the group consisting of H, D, F, Cl, Br, I, CHO, CN, N(Ar¹)₂, C(=O)Ar¹, P(=O)(Ar¹)₂, S(=O)Ar¹, S(=O)₂Ar¹ NO², Si(R⁵)₃, B(OR⁵)₂, OSO₂R⁵, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁵, where in each case one or more non-adjacent CH₂ groups may be replaced by R⁵C=CR⁵, C=C, Si(R⁵)₂, Ge(R⁵)₂, Sn(R⁵)₂, C=O, C=S, C=Se, P(=O)(R⁵), SO, SO₂, O, S or CONR⁵ and where one or more H atoms may be replaced by D, F, Cl, Br, I, CN or NO², an aromatic or heteroaromatic ring system having 5 to 40 aromatic ring atoms, which may in each case be substituted by one or more radicals R⁵, an aryloxy or heteroaryloxy group having 5 to 40 aromatic ring atoms, which may be substituted by one or more radicals R⁵, a thioaryl or thio-heteroaryl group having 5 to 40 aromatic ring atoms, which may be substituted by one or more radicals R⁵, where two or more adjacent substituents R⁴ may optionally form a mono- or polycyclic, aliphatic ring system or aromatic ring system, which may be substituted by one or more radicals R⁵;
   R⁵ is selected on each occurrence, identically or differently, from the group consisting of H, D, F, Cl, Br, I, CN, a straight-chain alkyl, alkoxy or thioalkyl group having 1 to 40 C atoms or a branched or cyclic alkyl, alkoxy or thioalkyl group having 3 to 40 C atoms, where in each case one or more non-adjacent CH₂ groups may be replaced by SO, SO₂, O, S and where one or more H atoms may be replaced by D, F, Cl, Br or I, an aromatic or heteroaromatic ring system having 5 to 60 aromatic ring atoms;
   Ar, Ar¹ is on each occurrence, identically or differently, an aromatic or heteroaromatic ring system having 5 to 40 aromatic ring atoms, which may in each case also be substituted by one or more radicals R⁴;
   m, n, r are, identically or differently, 0 or 1;
   p is 3;
   q, u are each 4;
   s is 3 or 4, where s + r = 4;
   t is 4 or 5, where t + r = 5.

For the purposes of the present application, the following definitions of chemical groups apply:
An aryl group in the sense of this invention contains 6 to 60 C atoms in the ring system, which means that it contains 6 to 60 aromatic ring atoms which all stand for C; a heteroaryl group in the sense of this invention contains 5 to 60 aromatic ring atoms, at least one of which is a heteroatom. The heteroatoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group (-O-aryl) in accordance with the definition of the present invention is taken to mean an aryl group, as defined above, which is bonded via an oxygen atom. An analogous definition applies to heteroaryloxy groups.

An thioaryl group (-S-aryl) in accordance with the definition of the present invention is taken to mean an aryl group, as defined above, which is bonded via a sulfur atom. An analogous definition applies to thio-heteroaryl groups.

An aromatic ring system in the sense of this invention contains 6 to 60 C atoms in the ring system. A heteroaromatic ring system in the sense of this invention contains 5 to 60 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5-60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-tri-fluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoro-ethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following schemes:

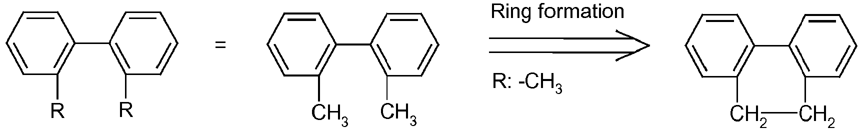

Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

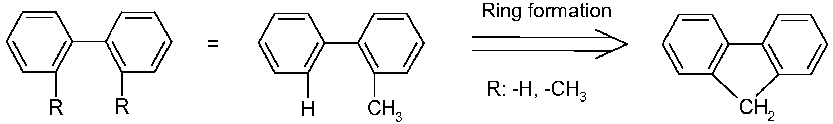

According to a preferred embodiment, m + n = 0 or 1. More preferably, m + n = 0.

It is particularly preferred that X is selected from the group consisting of C(R⁰)₂, O, S, and SO₂ and r is equal to 0.

It is very particularly preferred that X is C(R⁰)₂ and r is equal to 0.

The group Ar is preferably, identically or differently on each occurrence, selected from aromatic or heteroaromatic ring systems having 5 to 18 aromatic ring atoms, which may in each case also be substituted by one or more radicals R⁴.

The groups Ar here is very preferably selected, identically or differently on each occurrence, from the groups of the following formulae (10) to (66), 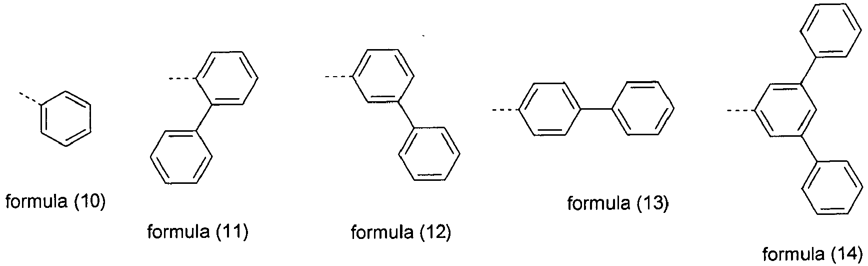 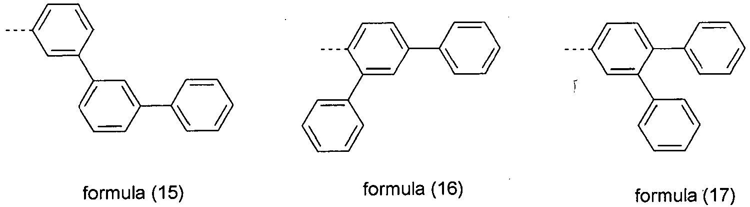 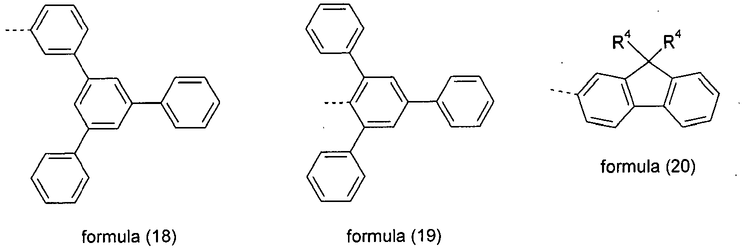 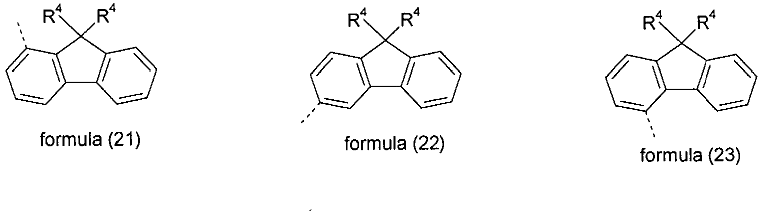 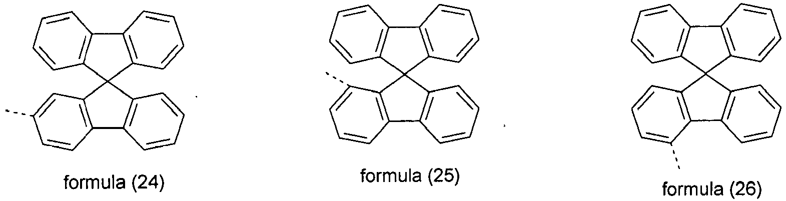 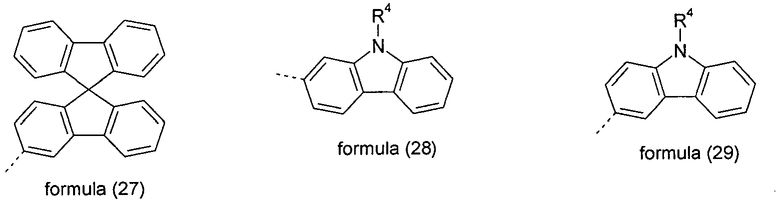 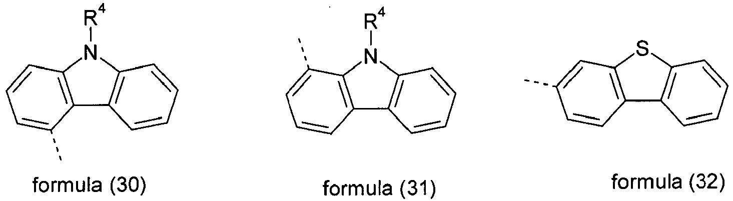 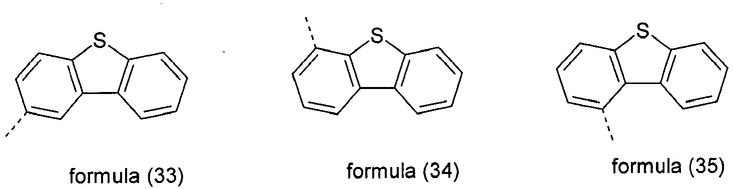 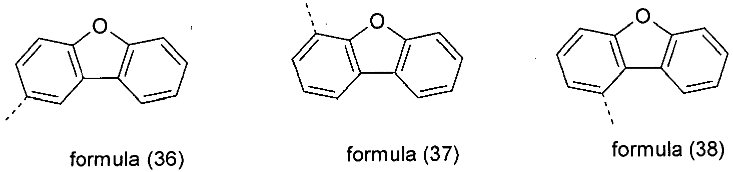 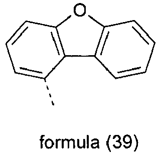 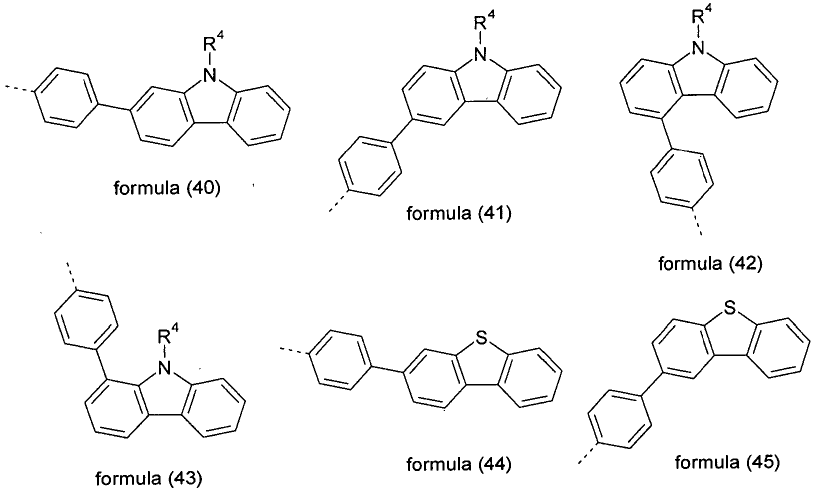 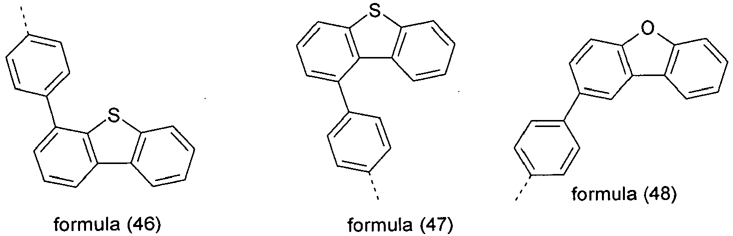 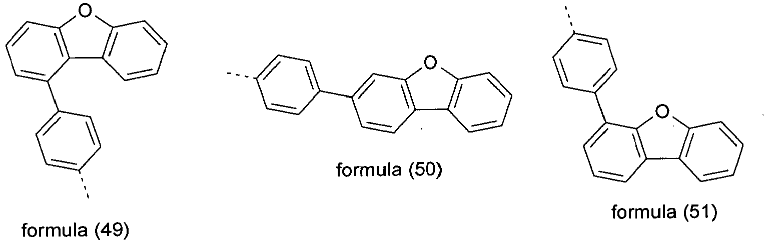 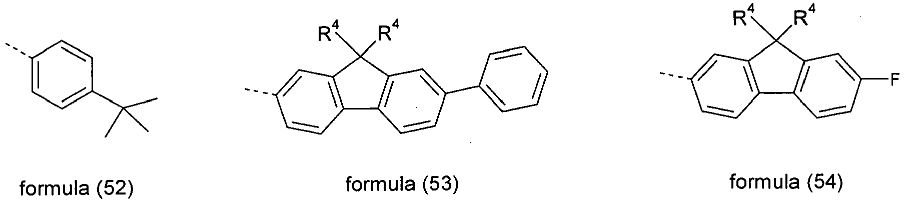 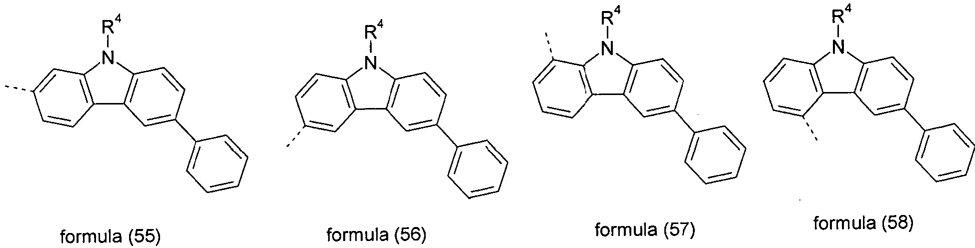 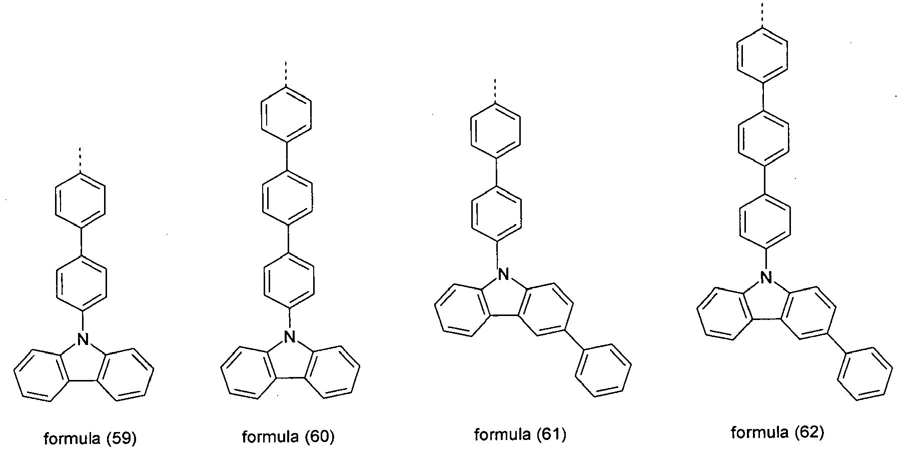 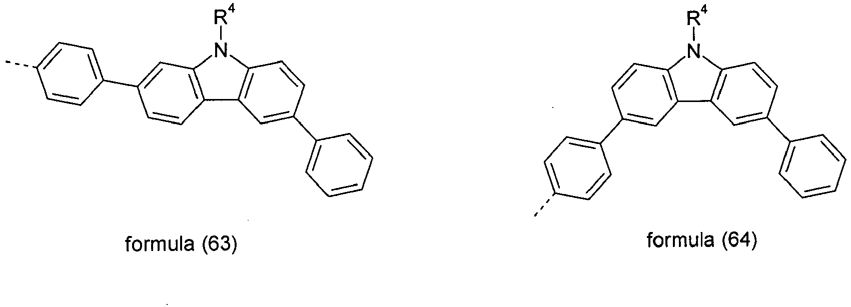 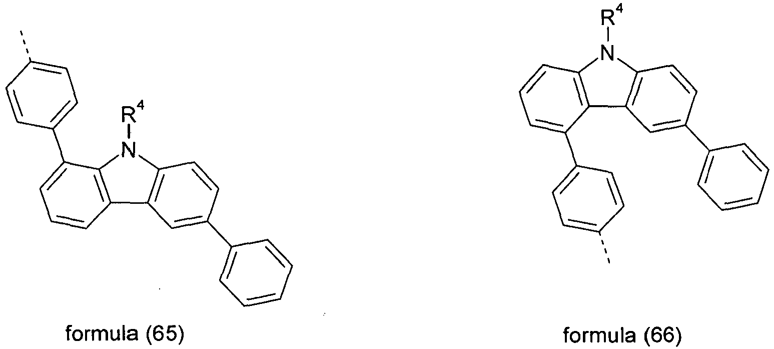 where the dashed bond indicates the bond to the nitrogen, and the groups may be substituted by one or more radicals R⁴, but are preferably unsubstituted.

In a further preferred embodiment, the group N(Ar)₂ is a group A of formula (2).

In a preferred embodiment of the invention, the compound of the formula (1) is selected from the compounds of the following formulae (1-1) to (1-6),

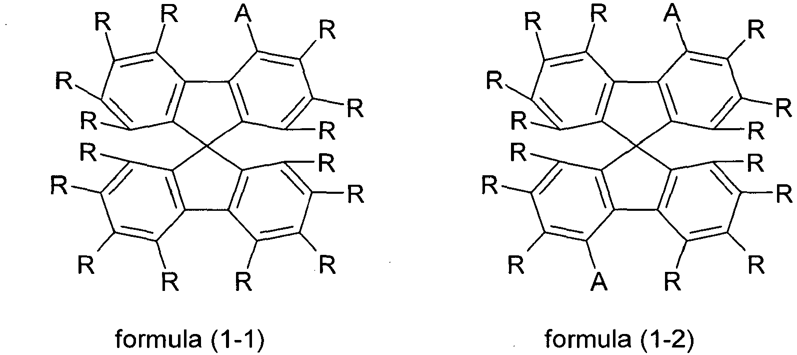

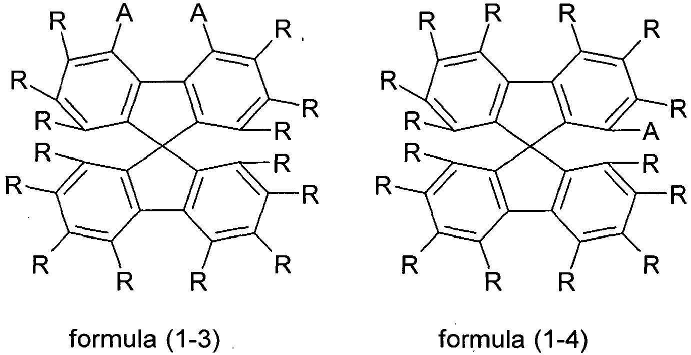

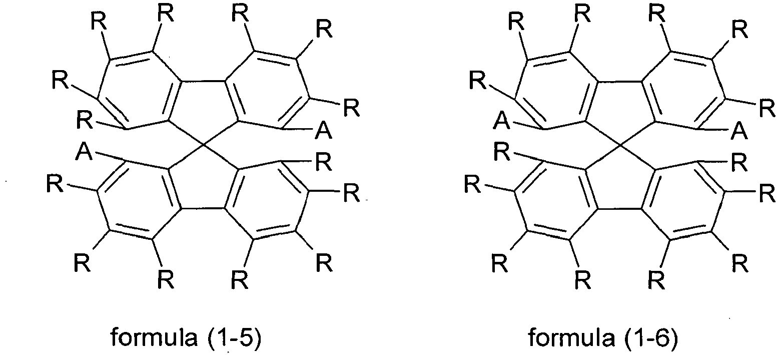

Formulae (1-1) and (1-4) are particularly preferred among formulae (1-1) to (1-6).

In a particularly preferred embodiment of the invention, the compounds of formulae (1-1) to (1-6) are selected from the compounds of the following formulae (1-1a) to (1-6a), 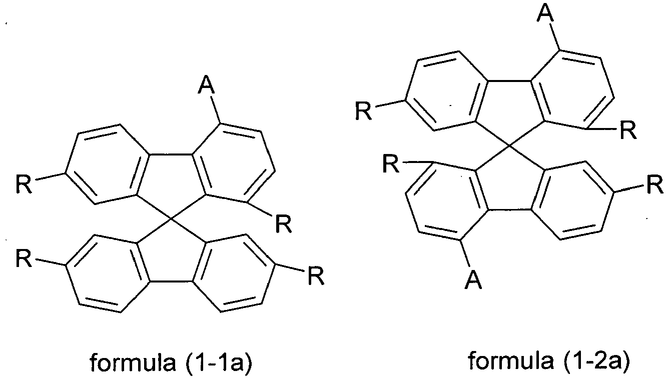 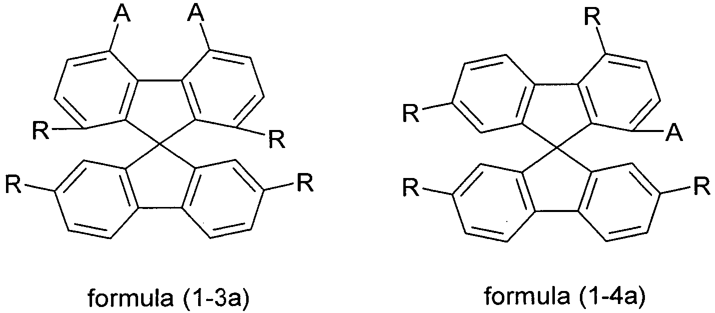 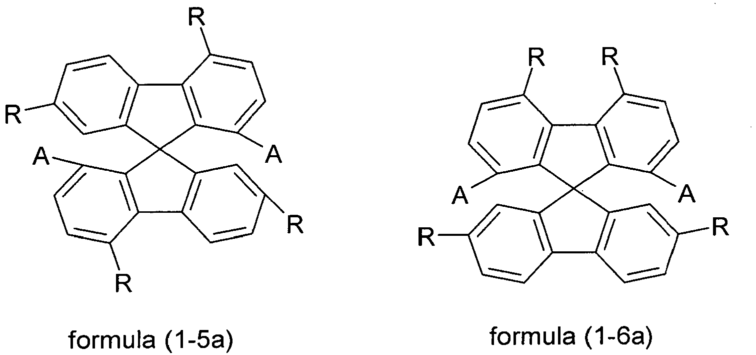 where the symbols and indices used have the meanings as indicated above.

Formulae (1-1a) and (1-4a) are particularly preferred among formulae (1-1a) to (1-6a).

In a very particularly preferred embodiment of the invention, the compounds of formulae (1-1a) to (1-6a) are selected from the compounds of the following formulae (1-1b) to (1-6b), 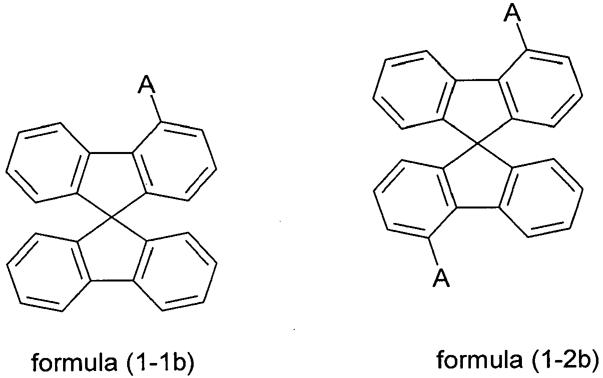 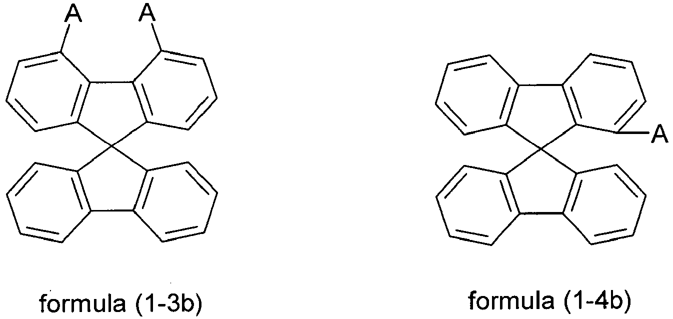 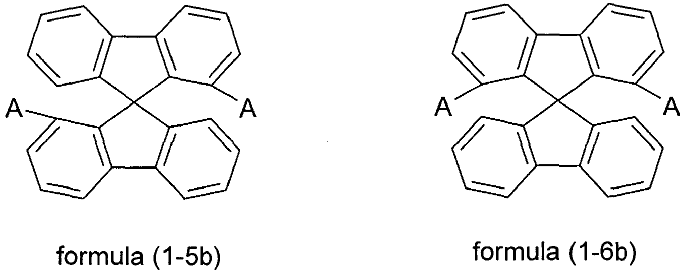 where the symbol A has the same meanings as indicated above.

Formulae (1-1b) and (1-4b) are particularly preferred among formulae (1-1b) to (1-6b).

In a preferred embodiment of the invention, R is selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R⁴.

In a particularly preferred embodiment of the invention, R is selected on each occurrence, identically or differently, from the group consisting of H, D, F, CN, a straight-chain alkyl group having 1 to 5 C atoms or a branched or cyclic alkyl group having 3 to 6 C atoms, an aryl or heteroaryl group having 5 to 18 aromatic ring atoms, which may in each case be substituted by one or more radicals R⁸.

R in the compounds of the formulae (1), (1-1) to (1-6) and (1-1a) to (1-6a) are very particularly preferably selected, identically or differently on each occurrence, from the group consisting of H, F, CN, methyl, tert-Butyl or phenyl.

It is preferred that R¹ and R³ are selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R⁴.

It is further preferred that R² is selected on each occurrence, identically or differently, from the group consisting of H, D, F, CN, a straight-chain alkyl, or alkoxy group having 1 to 10 C atoms or a branched or cyclic alkyl or alkoxy group having 3 to 10 C atoms, each of which may be substituted by one or more radicals R⁴, where in each case one or more non-adjacent CH₂ groups may be replaced by O or S and where one or more H atoms may be replaced by F, an aryl or heteroaryl ring system having 5 to 24 aromatic ring atoms, which may in each case be substituted by one or more radicals R⁴, an aryloxy or heteroaryloxy group having 5 to 24 aromatic ring atoms, which may be substituted by one or more radicals R⁴, a thioaryl or thio-heteroaryl group having 5 to 24 aromatic ring atoms, which may be substituted by one or more radicals R⁴, where two or more adjacent substituents R², may optionally form a mono- or polycyclic, aliphatic ring system or aromatic ring system, which may be substituted by one or more radicals R⁴.

According to a particularly preferred embodiment, two or more adjacent substituents R² do not form a mono- or polycyclic, aliphatic ring system or aromatic ring system.

According to a further particularly preferred embodiment, two or more adjacent substituents R² do form a mono- or polycyclic, aliphatic ring system or aromatic ring system.

It is very particularly preferred that two adjacent substituents R² form a ring according to one of the routes (1) to (5):

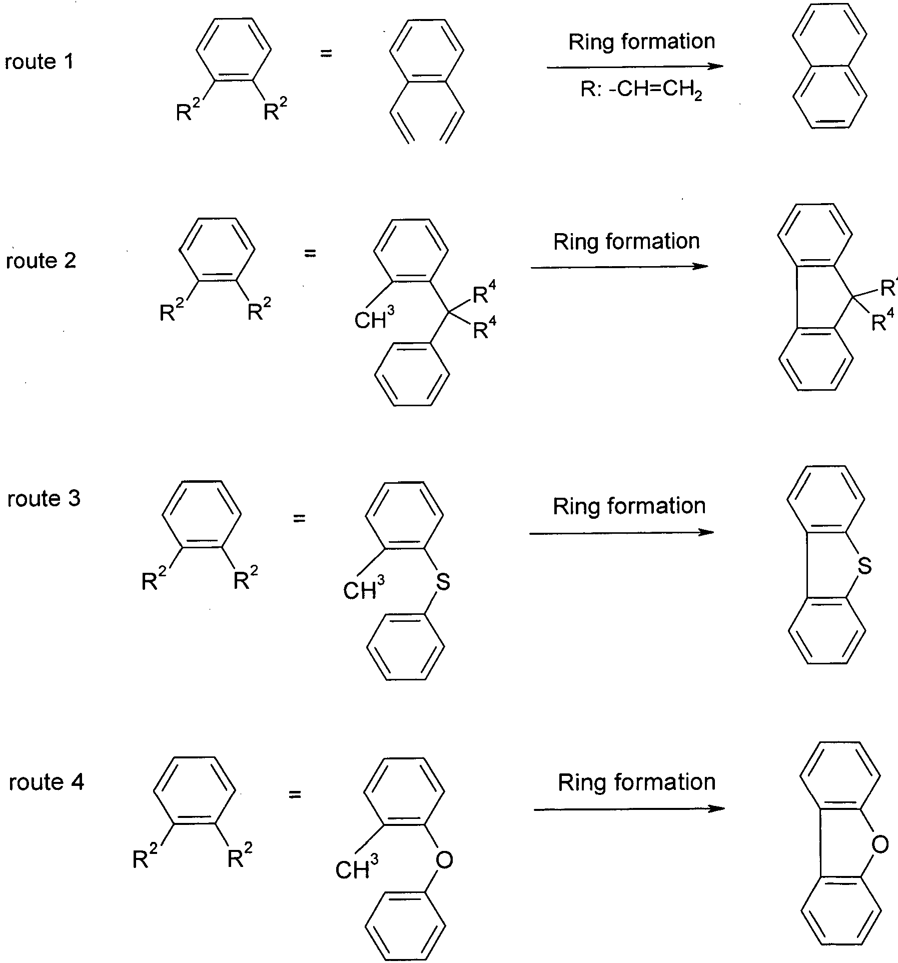

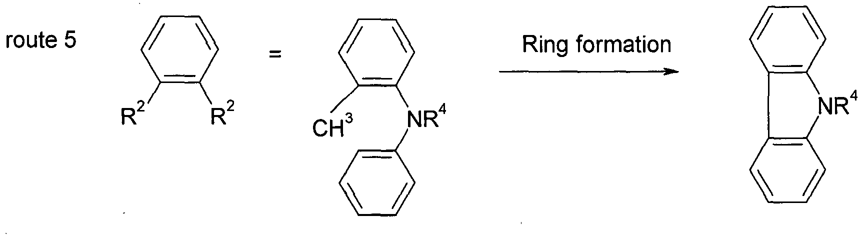

In a further preferred embodiment of the invention, R⁰ is selected on each occurrence, identically or differently, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where in each case one or more non-adjacent CH₂ groups may be replaced by O and where one or more H atoms may be replaced by D, F or CN, an aromatic or heteroaromatic ring system having 5 to 14 aromatic ring atoms, which may in each case be substituted by one or more radicals R⁴, where two substituents R⁰ may optionally form a monocyclic aliphatic ring system, which may be substituted by one or more radicals R⁴.

R⁰ is particularly preferably selected on each occurrence, identically or differently, from the group consisting of H, a straight-chain alkyl group having 1 to 10 C atoms or a branched or cyclic alkyl group having 3 to 10 C atoms, each of which may be substituted by one or more radicals R⁴.

For compounds which are processed by vacuum evaporation, the alkyl groups preferably have not more than four C atoms, particularly preferably not more than 1 C atom. For compounds which are processed from solution, suitable compounds are also those which are substituted by linear, branched or cyclic alkyl groups having up to 10 C atoms or which are substituted by oligoarylene groups, for example ortho-, meta-, para- or branched terphenyl or quaterphenyl groups.

Examples of suitable compounds according to the invention are the compounds shown in the following table:

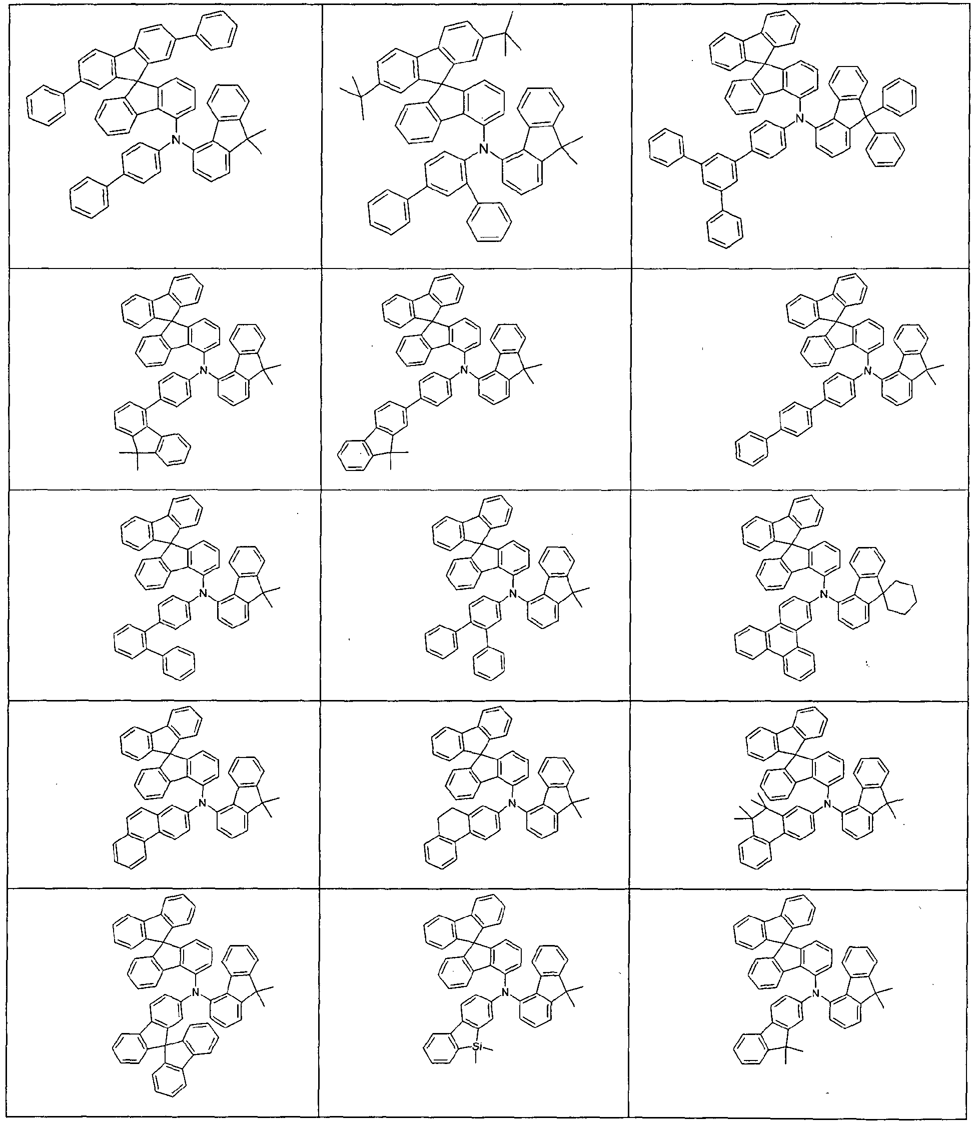

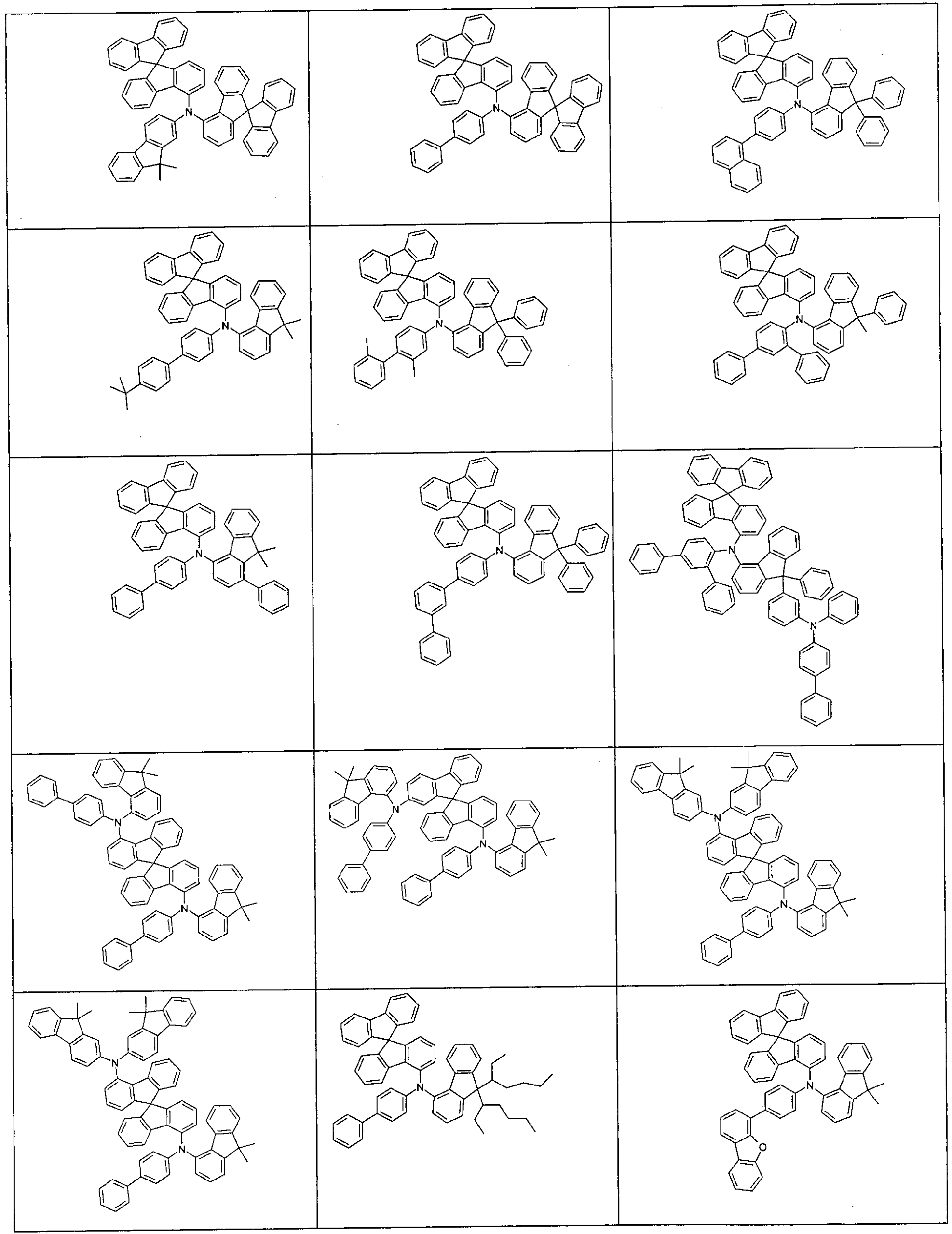

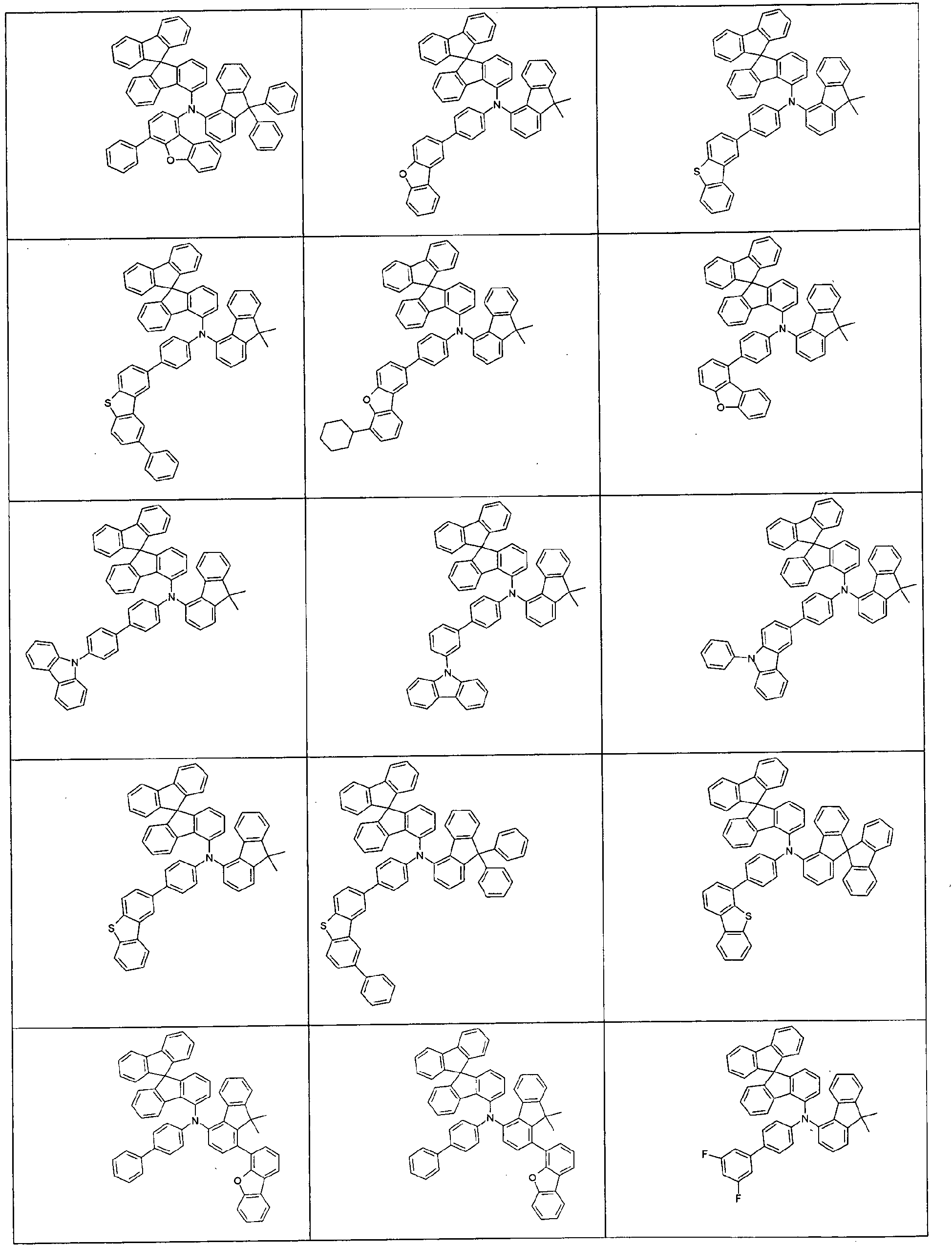

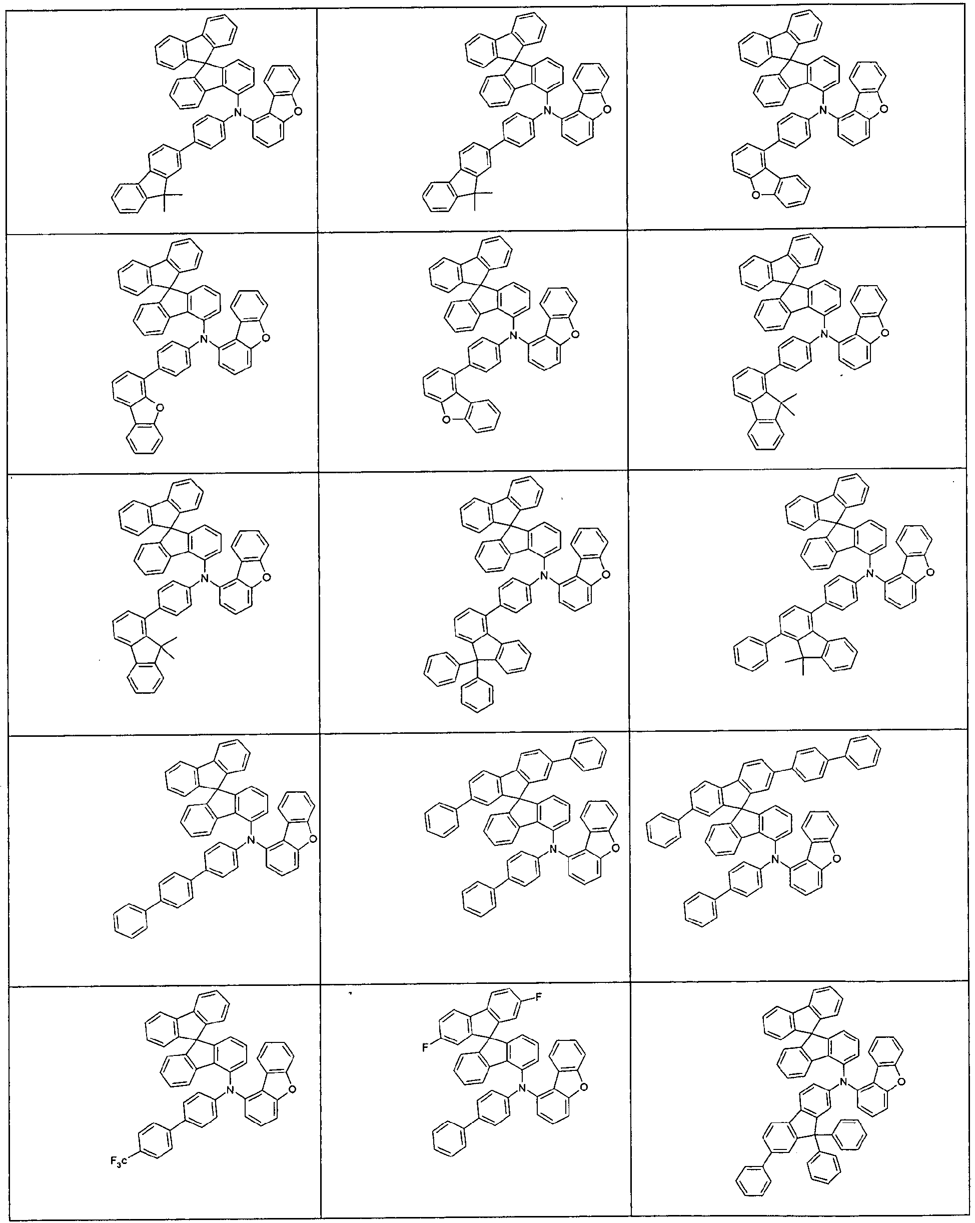

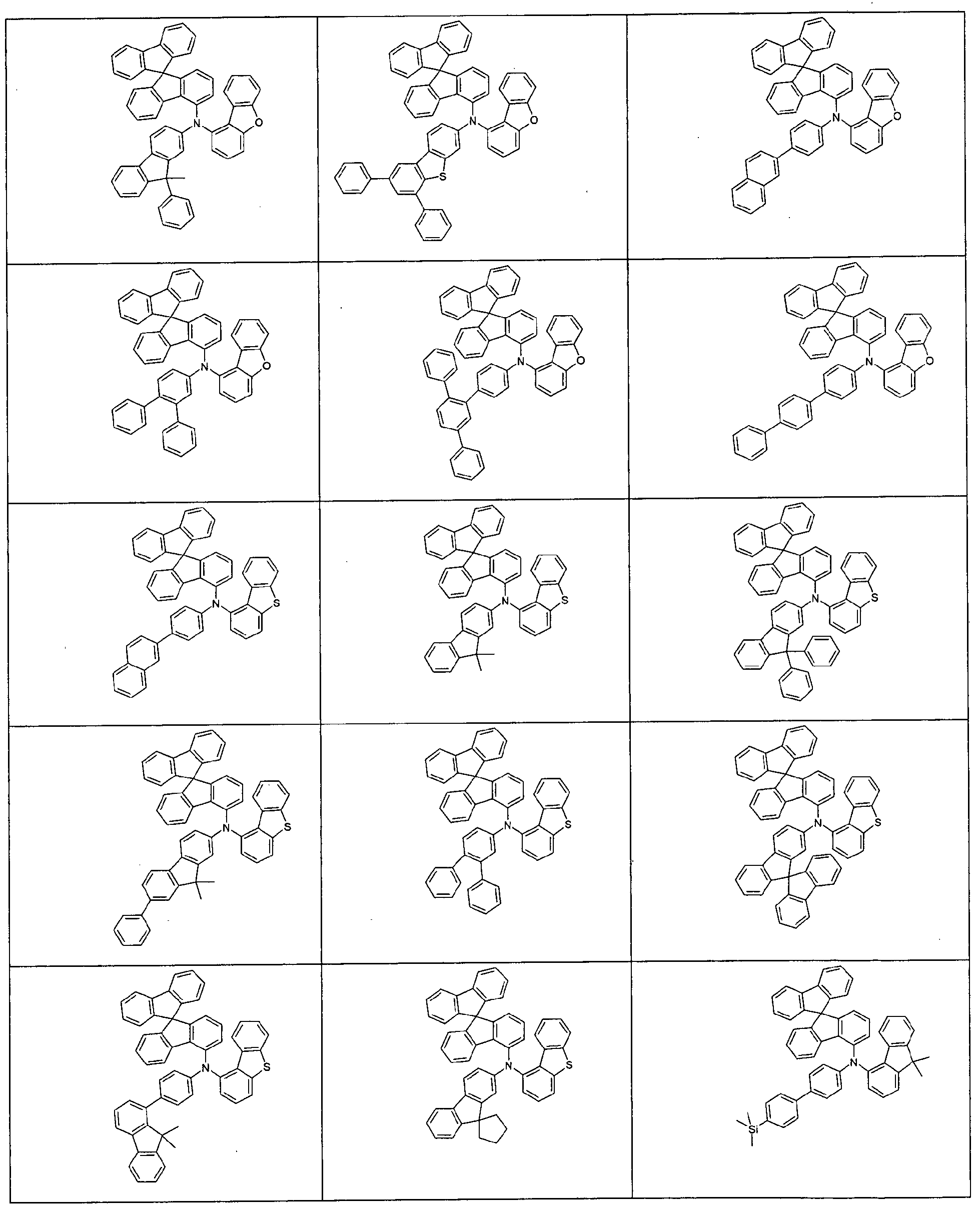

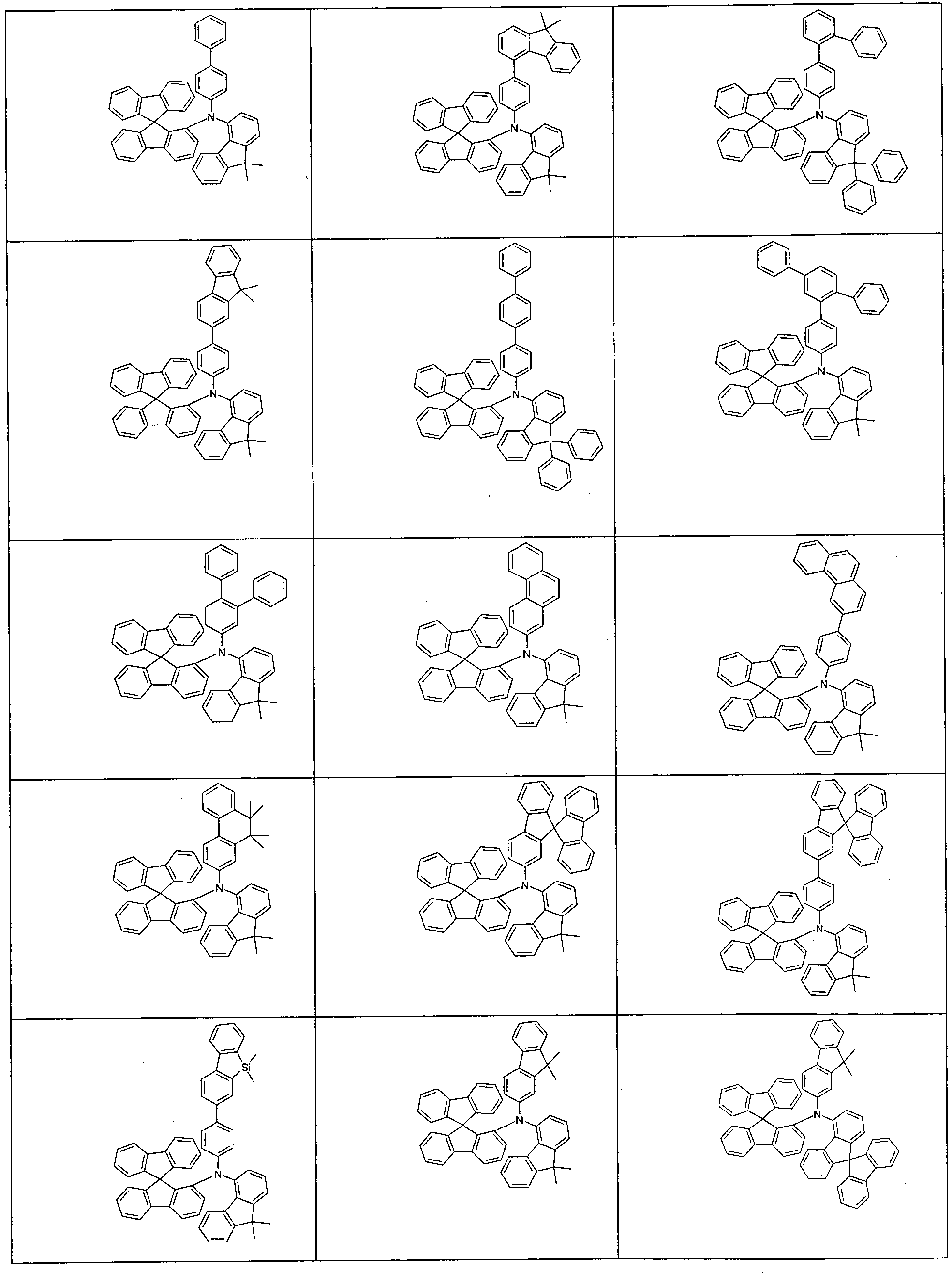

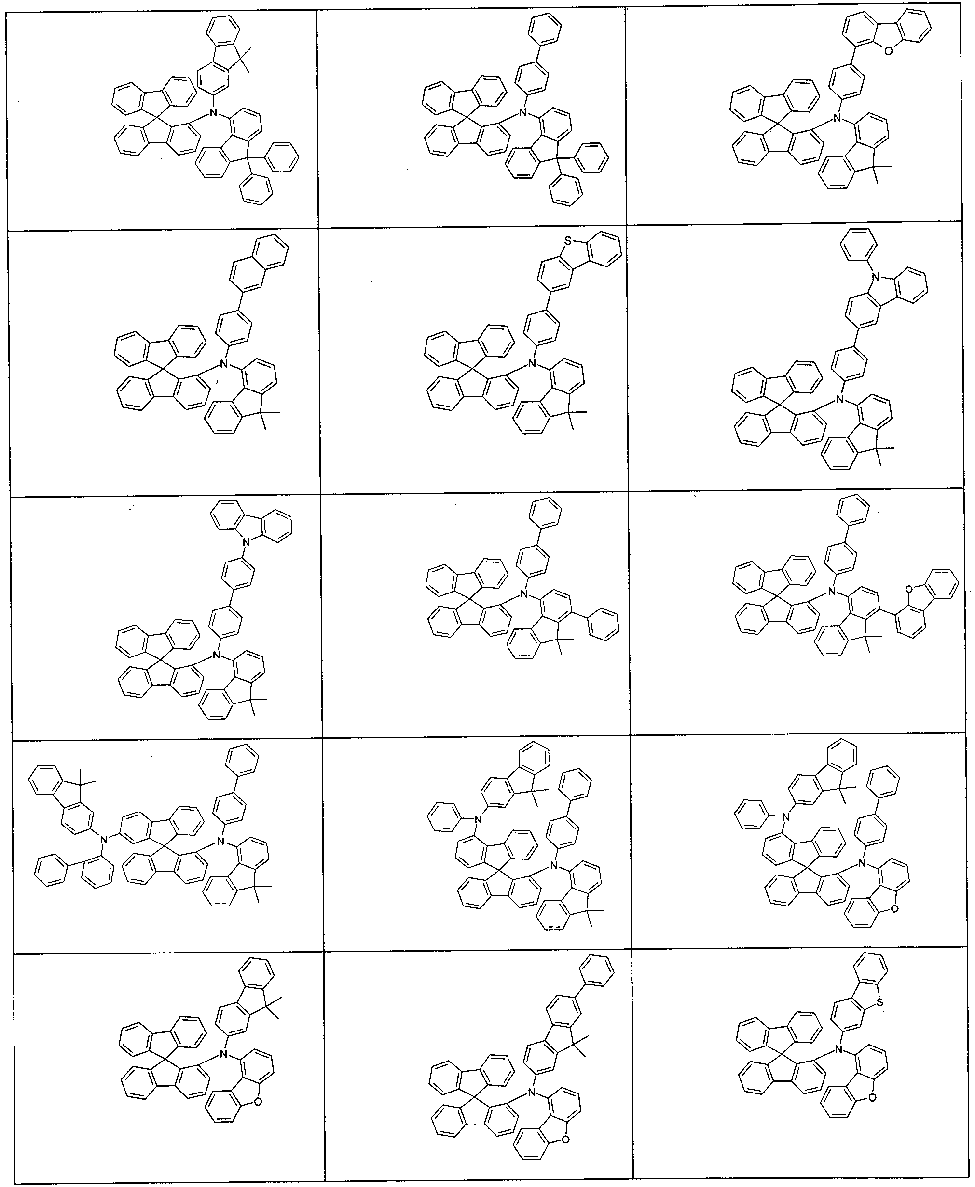

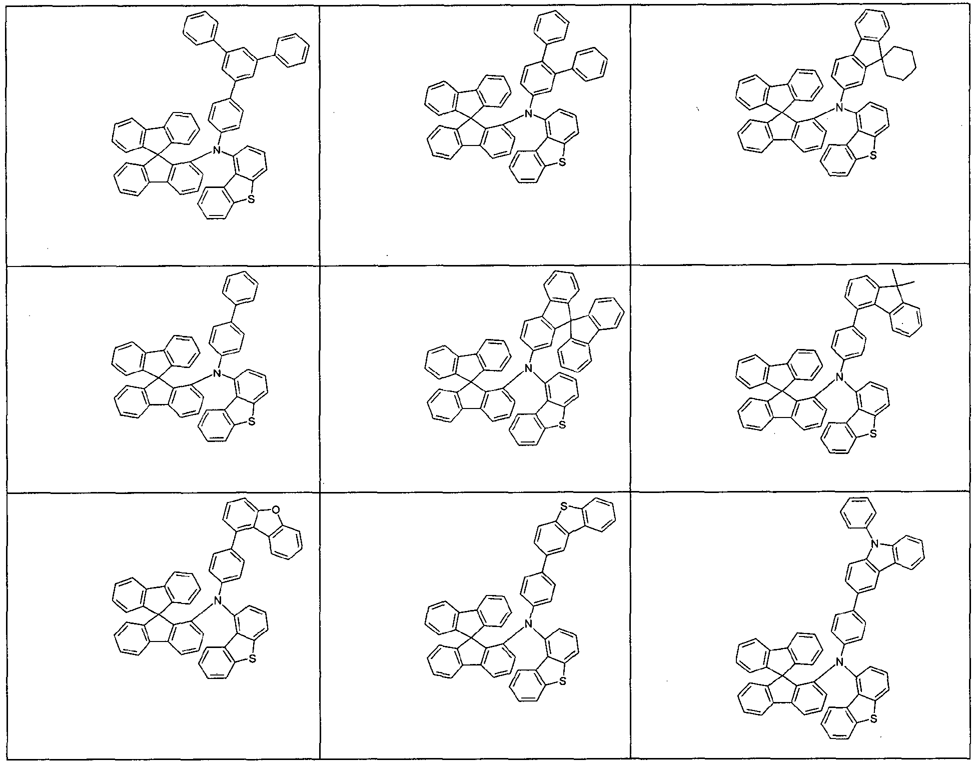

The compounds according to the invention can be prepared by synthetic steps known to the person skilled in the art, such as, for example, bromination, Ullmann arylation, Hartwig-Buchwald coupling, etc.

The synthesis of compounds according to the invention is shown in Scheme 1, where two different access routes are shown.
The syntheses generally start from the 1- or 4-halogenated, in particular brominated, spirobifluorene derivatives. These syntheses are already described in WO 2013/120577. The introduction of the diarylamino group can be carried out in one step by a C-N coupling reaction coupling reaction using a secondary diarylamine as starting material or in two steps using sequentially a primary diarylamine and an aryl-bromide for the first and second C-N coupling respectively.
Analogously, another suitable leaving group, for example tosylate or triflate, can be used instead of the halogen. Likewise, corresponding substituted structures can also be synthesised entirely analogously.
As example for the C-N coupling reactions a Hartwig-Buchwald coupling or an Ullmann coupling can be used.

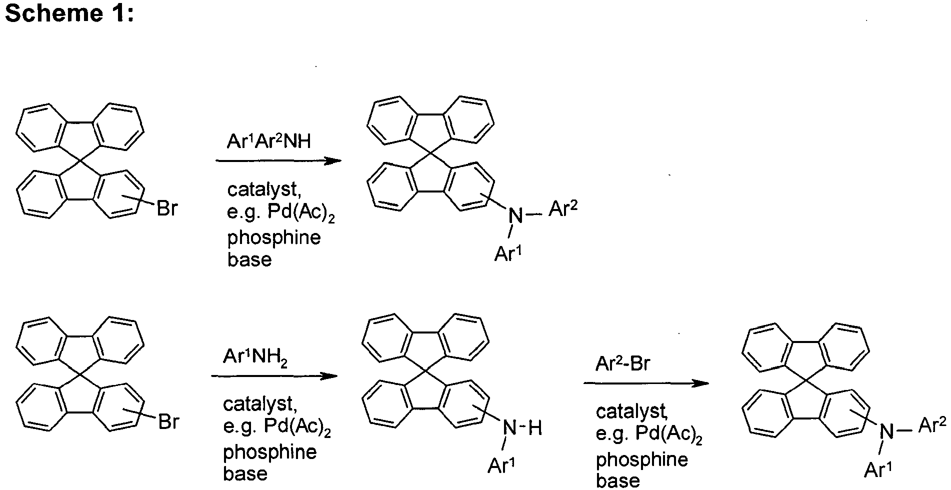

The present invention therefore furthermore relates to a process for the preparation of a compound of the formula (1), characterised in that the diarylamino group is introduced by C-N coupling reactions between a 1- or 4-halogenated spirobifluorene and an arylamine.

The compounds according to the invention described above, in particular compounds which are substituted by reactive leaving groups, such as bromine, iodine, boronic acid or boronic acid ester, can be used as monomers for the preparation of corresponding oligomers, dendrimers or polymers. The oligomerisation or polymerisation here is preferably carried out via the halogen functionality or the boronic acid functionality.

Considered herein are furthermore oligomers, polymers or dendrimers comprising one or more compounds of the formula (1), where the bond(s) to the polymer, oligomer or dendrimer may be localised at any desired positions in formula (1) substituted by R. Depending on the linking of the compound of the formula (1), the compound is part of a side chain of the oligomer or polymer or part of the main chain. An oligomer taken to mean a compound which is built up from at least three monomer.A polymer is taken to mean a compound which is built up from at least ten monomer units. The polymers, oligomers or dendrimers considered herein may be conjugated, partially conjugated or non-conjugated. They may be linear, branched or dendritic. In the structures linked in a linear manner, the units of the formula (1) may be linked directly to one another or linked to one another via a divalent group, for example via a substituted or unsubstituted alkylene group, via a heteroatom or via a divalent aromatic or heteroaromatic group. In branched and dendritic structures, three or more units of the formula (1) may, for example, be linked via a trivalent or polyvalent group, for example via a trivalent or polyvalent aromatic or heteroaromatic group, to give a branched or dendritic oligomer or polymer. The same preferences as described above for compounds of the formula (1) apply to the recurring units of the formula (1) in oligomers, dendrimers and polymers.

For the preparation of the oligomers or polymers, the monomers according to the invention are homopolymerised or copolymerised with further monomers. Suitable and preferred comonomers are selected from fluorenes (for example in accordance with EP 842208 or WO 00/22026), spirobifluorenes (for example in accordance with EP 707020, EP 894107 or WO 06/061181), para-phenylenes (for example in accordance with WO 92/18552), carbazoles (for example in accordance with WO 04/070772 or WO 04/113468), thiophenes (for example in accordance with EP 1028136), dihydrophenanthrenes (for example in accordance with WO 05/014689 or WO 07/006383), cis- and trans-indenofluorenes (for example in accordance with WO 04/041901 or WO 04/113412), ketones (for example in accordance with WO 05/040302), phenanthrenes (for example in accordance with WO 05/104264 or WO 07/017066) or also a plurality of these units. The polymers, oligomers and dendrimers usually also contain further units, for example emitting (fluorescent or phosphorescent) units, such as, for example, vinyltriarylamines (for example in accordance with WO 07/068325) or phosphorescent metal complexes (for example in accordance with WO 06/003000), and/or charge-transport units, in particular those based on triarylamines.

The polymers, oligomers and dendrimers have advantageous properties, in particular long lifetimes, high efficiencies and good colour coordinates.

The polymers and oligomers are generally prepared by polymerisation of one or more types of monomer, at least one monomer of which results in recurring units of the formula (1) in the polymer. Suitable polymerisation reactions are known to the person skilled in the art and are described in the literature. Particularly suitable and preferred polymerisation reactions which result in C-C or C-N links are the following:
(A) SUZUKI polymerisation;
(B) YAMAMOTO polymerisation;
(C) STILLE polymerisation; and
(D) HARTWIG-BUCHWALD polymerisation.
The way in which the polymerisation can be carried out by these methods and the way in which the polymers can then be separated off from the reaction medium and purified is known to the person skilled in the art and is described in detail in the literature, for example in WO 2003/048225, WO 2004/037887 and WO 2004/037887.

Considered herein is thus also a process for the preparation of the polymers, oligomers and dendrimers according to the invention, which is characterised in that they are prepared by SUZUKI polymerisation, YAMAMOTO polymerisation, STILLE polymerisation or HARTWIG-BUCHWALD polymerisation. The dendrimers can be prepared by processes known to the person skilled in the art or analogously thereto. Suitable processes are described in the literature, such as, for example, in Frechet, Jean M. J.; Hawker, Craig J., "Hyper-branched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 02/067343 A1 and WO 2005/026144 A1.

The compounds according to the invention are suitable for use in an electronic device. An electronic device here is taken to mean a device which comprises at least one layer which comprises at least one organic compound. However, the component here may also comprise inorganic materials or also layers built up entirely from inorganic materials.
The present invention therefore furthermore relates to the use of the compounds according to the invention in an electronic device, in particular in an organic electroluminescent device.
The present invention still furthermore relates to an electronic device comprising at least one compound according to the invention. The preferences stated above likewise apply to the electronic devices.

The electronic device is preferably selected from the group consisting of organic electroluminescent devices (organic light-emitting diodes, OLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells (ODSSCs), organic optical detectors, organic photoreceptors, organic field-quench devices (O-FQDs), light-emitting electrochemical cells (LECs), organic laser diodes (O-lasers) and organic plasmon emitting devices (D. M. Koller et al., Nature Photonics 2008, 1-4), but preferably organic electroluminescent devices (OLEDs), particularly preferably phosphorescent OLEDs.

The organic electroluminescent devices and the light-emitting electrochemical cells can be employed for various applications, for example for monochromatic or polychromatic displays, for lighting applications or for medical and/or cosmetic applications, for example in phototherapy.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. Interlayers, which have, for example, an exciton-blocking function, may likewise be introduced between two emitting layers. However, it should be pointed out that each of these layers does not necessarily have to be present.

The organic electroluminescent device here may comprise one emitting layer or a plurality of emitting layers. If a plurality of emission layers is present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). It is possible here for all emitting layers to be fluorescent or for all emitting layers to be phosphorescent or for one or more emitting layers to be fluorescent and one or more other layers to be phosphorescent.

The compound according to the invention in accordance with the embodiments indicated above can be employed here in different layers, depending on the precise structure. Preference is given to an organic electroluminescent device comprising a compound of the formula (1) or the preferred embodiments as hole-transport material in a hole-transport or hole-injection or exciton-blocking layer or as matrix material for fluorescent or phosphorescent emitters, in particular for phosphorescent emitters. The preferred embodiments indicated above also apply to the use of the materials in organic electronic devices.

In a preferred embodiment of the invention, the compound of the formula (1) or the preferred embodiments is employed as hole-transport or hole-injection material in a hole-transport or hole-injection layer. The emitting layer here can be fluorescent or phosphorescent. A hole-injection layer in the sense of the present invention is a layer which is directly adjacent to the anode. A hole-transport layer in the sense of the present invention is a layer which is located between a hole-injection layer and an emitting layer.

In still a further preferred embodiment of the invention, the compound of the formula (1) or the preferred embodiments is employed in an exciton-blocking layer. An exciton-blocking layer is taken to mean a layer which is directly adjacent to an emitting layer on the anode side.

The compound of the formula (1) or the preferred embodiments is particularly preferably employed in a hole-transport or exciton-blocking layer.

If the compound of the formula (1) is employed as a hole-transport material in a hole-tranport layer, a hole-injection layer or an exciton-blocking layer, then the compound of formula (1) can be used in such a layer as a single material, i.e. in a proportion of 100%, or the compound of formula (1) can be used in combination with one or more further compounds in such a layer. According to a preferred embodiment, the organic layer comprising the compound of formula (1) additionally comprises one or more p-dopants. Preferred p-dopant for the present invention are organic compounds that can accept electrons (electron acceptors) and can oxidize one or more of the other compounds present in the mixture.

Particularly preferred embodiments of p-dopants are described in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 and DE 102012209523.

Particularly preferred as p-dopants are quinodimethane compounds, azaindenofluorendione, azaphenalene, azatriphenylene, I₂, metal halides, preferably transition metal halides, metal oxides, preferably metal oxides containing at least one transition metal or a metal of the 3rd main group and transition metal complexes, preferably complexes of Cu, Co, Ni, Pd and Pt with ligands containing at least one oxygen atom as binding site. Also preferred are transition metal oxides as dopants, preferably oxides of rhenium, molybdenum and tungsten, particularly preferably Re₂O₇, MoO₃, WO₃ and ReO₃.

The p-dopants are preferably distributed substantially uniformly in the p-doped layers. This can be achieved for example by co-evaporation of the p-dopant and of the hole-transport material matrix.

Particularly preferred p-dopants are selected from the compounds (D-1) to (D-13):

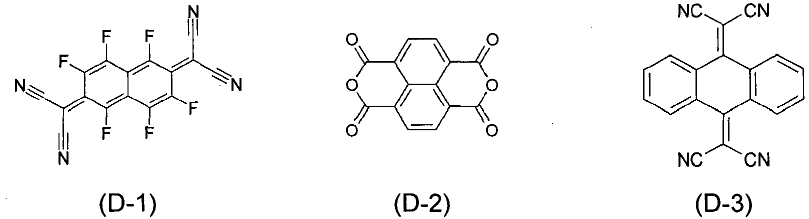

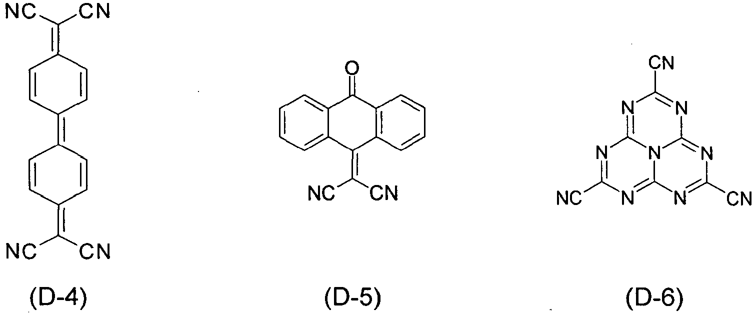

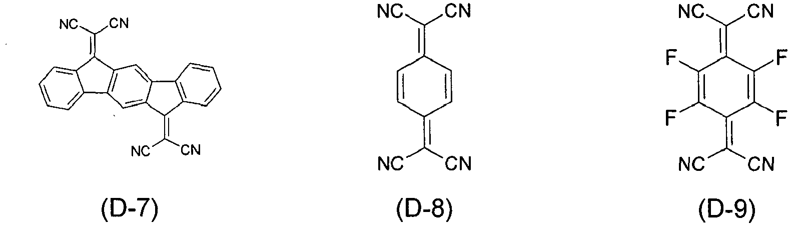

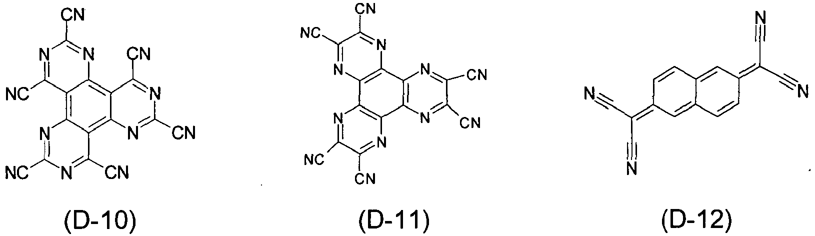

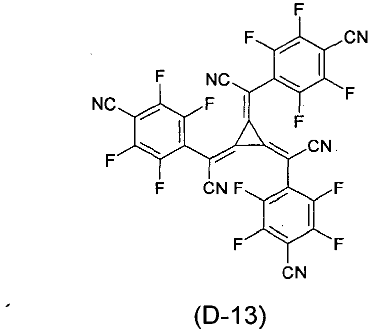

In an embodiment of the invention, the compound of the formula (1) or the preferred embodiments is used in a hole-transport or -injection layer in combination with a layer which comprises a hexaazatriphenylene derivative, in particular hexacyanohexaazatriphenylene (for example in accordance with EP 1175470). Thus, for example, preference is given to a combination which looks as follows: anode - hexaazatriphenylene derivative-hole-transport layer, where the hole-transport layer comprises one or more compounds of the formula (1) or the preferred embodiments. It is likewise possible in this structure to use a plurality of successive hole-transport layers, where at least one hole-transport layer comprises at least one compound of the formula (1) or the preferred embodiments. A further preferred combination looks as follows: anode - hole-transport layer - hexaazatriphenylene derivative - hole-transport layer, where at least one of the two hole-transport layers comprises one or more compounds of the formula (1) or the preferred embodiments. It is likewise possible in this structure to use a plurality of successive hole-transport layers instead of one hole-transport layer, where at least one hole-transport layer comprises at least one compound of the formula (1) or the preferred embodiments.

In a further preferred embodiment of the invention, the compound of the formula (1) or the preferred embodiments is employed as matrix material for a fluorescent or phosphorescent compound, in particular for a phosphorescent compound, in an emitting layer. The organic electroluminescent device here may comprise one emitting layer or a plurality of emitting layers, where at least one emitting layer comprises at least one compound according to the invention as matrix material.

If the compound of the formula (1) or the preferred embodiments is employed as matrix material for an emitting compound in an emitting layer, it is preferably employed in combination with one or more phosphorescent materials (triplet emitters). Phosphorescence in the sense of this invention is taken to mean the luminescence from an excited state having a spin multiplicity > 1, in particular from an excited triplet state. For the purposes of this application, all luminescent complexes containing transition metals or lanthanoids, in particular all luminescent iridium, platinum and copper complexes, are to be regarded as phosphorescent compounds.

The mixture comprising the matrix material, which comprises the compound of the formula (1) or the preferred embodiments, and the emitting compound comprises between 99.9 and 1% by weight, preferably between 99 and 10% by weight, particularly preferably between 97 and 60% by weight, in particular between 95 and 80% by weight, of the matrix material, based on the entire mixture comprising emitter and matrix material. Correspondingly, the mixture comprises between 0.1 and 99% by weight, preferably between 1 and 90% by weight, particularly preferably between 3 and 40% by weight, in particular between 5 and 20% by weight, of the emitter, based on the entire mixture comprising emitter and matrix material. The limits indicated above apply, in particular, if the layer is applied from solution. If the layer is applied by vacuum evaporation, the same numerical values apply, with the percentage in this case being indicated in % by vol. in each case.

A particularly preferred embodiment of the present invention is the use of the compound of the formula (1) or the preferred embodiments as matrix material for a phosphorescent emitter in combination with a further matrix material. Particularly suitable matrix materials which can be employed in combination with the compounds of the formula (1) or the preferred embodiments are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-biscarbazolylbiphenyl), m-CBP or the carbazole derivatives disclosed in WO 2005/039246, US 2005/0069729, JP 2004/288381, EP 1205527 or WO 2008/086851, indolocarbazole derivatives, for example in accordance with WO 2007/063754 or WO 2008/056746, indenocarbazole derivatives, for example in accordance with WO 2010/136109 or WO 2011/000455, azacarbazole derivatives, for example in accordance with EP 1617710, EP 1617711, EP 1731584, JP 2005/347160, bipolar matrix materials, for example in accordance with WO 2007/137725, silanes, for example in accordance with WO 2005/111172, azaboroles or boronic esters, for example in accordance with WO 2006/117052, triazine derivatives, for example in accordance with WO 2010/015306, WO 2007/063754 or WO 08/056746, zinc complexes, for example in accordance with EP 652273 or WO 2009/062578, fluorene derivatives, for example in accordance with WO 2009/124627, diazasilole or tetraazasilole derivatives, for example in accordance with WO 2010/054729, diazaphosphole derivatives, for example in accordance with WO 2010/054730, or bridged carbazole derivatives, for example in accordance with US 2009/0136779, WO 2010/050778, WO 2011/042107 or WO 2011/088877. It is furthermore possible to use an electronically neutral co-host which has neither hole-transporting nor electron-transporting properties, as described, for example, in WO 2010/108579.

It is likewise possible to use two or more phosphorescent emitters in the mixture. In this case, the emitter which emits at shorter wavelength acts as co-host in the mixture.

Suitable phosphorescent compounds (= triplet emitters) are, in particular, compounds which emit light, preferably in the visible region, on suitable excitation and in addition contain at least one atom having an atomic number greater than 20, preferably greater than 38 and less than 84, particularly preferably greater than 56 and less than 80, in particular a metal having this atomic number. The phosphorescent emitters used are preferably compounds which contain copper, molybdenum, tungsten, rhenium, ruthenium, osmium, rhodium, iridium, palladium, platinum, silver, gold or europium, in particular compounds which contain iridium, platinum or copper.

Examples of the emitters described above are revealed by the applications WO 2000/70655, WO 2001/41512, WO 2002/02714, WO 2002/15645, EP 1191613, EP 1191612, EP 1191614, WO 2005/033244, WO 2005/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/157339 or WO 2012/007086. In general, all phosphorescent complexes as used in accordance with the prior art for phosphorescent OLEDs and as are known to the person skilled in the art in the area of organic electroluminescence are suitable, and the person skilled in the art will be able to use further phosphorescent complexes without inventive step.

In a further embodiment of the invention, the organic electroluminescent device according to the invention does not comprise a separate hole-injection layer and/or hole-transport layer and/or hole-blocking layer and/or electron-transport layer, i.e. the emitting layer is directly adjacent to the hole-injection layer or the anode, and/or the emitting layer is directly adjacent to the electron-transport layer or the electron-injection layer or the cathode, as described, for example, in WO 2005/053051. It is furthermore possible to use a metal complex which is identical or similar to the metal complex in the emitting layer as hole-transport or hole-injection material directly adjacent to the emitting layer, as described, for example, in WO 2009/030981.

It is furthermore possible to use the compound of the formula (1) or the preferred embodiments both in a hole-transport layer or exciton-blocking layer and as matrix in an emitting layer.

In the further layers of the organic electroluminescent device according to the invention, it is possible to use all materials as usually employed in accordance with the prior art. The person skilled in the art will therefore be able, without inventive step, to employ all materials known for organic electroluminescent devices in combination with the compounds of the formula (1) according to the invention or the preferred embodiments.

Preference is furthermore given to an organic electroluminescent device, characterised in that one or more layers are applied by means of a sublimation process, in which the materials are vapour-deposited in vacuum sublimation units at an initial pressure of usually less than 10⁻⁵ mbar, preferably less than 10⁻⁶ mbar. However, it is also possible for the initial pressure to be even lower, for example less than 10⁻⁷ mbar.

Preference is likewise given to an organic electroluminescent device, characterised in that one or more layers are applied by means of the OVPD (organic vapour phase deposition) process or with the aid of carrier-gas sublimation, in which the materials are applied at a pressure between 10⁻⁵ mbar and 1 bar. A special case of this process is the OVJP (organic vapour jet printing) process, in which the materials are applied directly through a nozzle and thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, LITI (light induced thermal imaging, thermal transfer printing), ink-jet printing, screen printing, flexographic printing, offset printing or nozzle printing. Soluble compounds, which are obtained, for example, by suitable substitution, are necessary for this purpose. These processes are also particularly suitable for the compounds according to the invention, since these generally have very good solubility in organic solvents.

Also possible are hybrid processes, in which, for example, one or more layers are applied from solution and one or more further layers are applied by vapour deposition. Thus, for example, the emitting layer can be applied from solution and the electron-transport layer by vapour deposition.

These processes are generally known to the person skilled in the art and can be applied by him without inventive step to organic electroluminescent devices comprising the compounds according to the invention.

The processing of the compounds according to the invention from the liquid phase, for example by spin coating or by printing processes, requires formulations of the compounds according to the invention. These formulations can be, for example, solutions, dispersions or mini-emulsions. It may be preferred to use mixtures of two or more solvents for this purpose. Suitable and preferred solvents are, for example, toluene, anisole, o-, m- or p-xylene, methyl benzoate, dimethylanisole, mesitylene, tetralin, veratrol, THF, methyl-THF, THP, chlorobenzene, dioxane or mixtures of these solvents.

The present invention therefore furthermore relates to a formulation, in particular a solution, dispersion or mini-emulsion, comprising at least one compound of the formula (1) or the preferred embodiments indicated above and at least one solvent, in particular an organic solvent. The way in which solutions of this type can be prepared is known to the person skilled in the art and is described, for example, in WO 2002/072714, WO 2003/019694 and the literature cited therein.

The present invention furthermore relates to mixtures comprising at least one compound of the formula (1) or the preferred embodiments indicated above and at least one further compound. The further compound can be, for example, a fluorescent or phosphorescent dopant if the compound according to the invention is used as matrix material. The mixture may then also additionally comprise a further material as additional matrix material.

The compounds according to the invention and the organic electroluminescent devices according to the invention are distinguished by the following surprising advantages over the prior art:
1. The compounds according to the invention are very highly suitable for use in a hole-transport or hole-injection layer in an organic electroluminescent device. They are also suitable, in particular, for use in a layer which is directly adjacent to a phosphorescent emitting layer, since the compounds according to the invention do not extinguish the luminescence.
2. The compounds according to the invention, employed as matrix material for fluorescent or phosphorescent emitters, result in very high efficiencies and long lifetimes. This applies, in particular, if the compounds are employed as matrix material together with a further matrix material and a phosphorescent emitter.
3. The compounds according to the invention, employed in organic electroluminescent devices, result in high efficiencies and in steep current/voltage curves with low use and operating voltages.
4. The compounds of the invention exhibit a high thermal stability and can therefore be sublimed without decomposition and without a residue.
5. The compounds of the invention have a low crystallinity and a high glass transition temperature, which leads to an improved lifetime.

These above-mentioned advantages are not accompanied by an impairment in the other electronic properties.

The invention is explained in greater detail by the following examples, without wishing to restrict it thereby. On the basis of the descriptions, the person skilled in the art will be able to carry out the invention throughout the range disclosed and prepare further compounds according to the invention without inventive step and use them in electronic devices or use the process according to the invention.

### Examples:

### A) Synthesis examples

The following syntheses are carried out under a protective-gas atmosphere, unless indicated otherwise. The starting materials can be purchased from ALDRICH or ABCR. The numbers in square brackets in the case of the starting materials known from the literature are the corresponding CAS numbers.

### Example 1:

### Synthesis of Bis-(9,9-spirobifluoren-4-yl)-(9,9-dimethyl-9H-fluoren-2-yl)-amine (1-1)

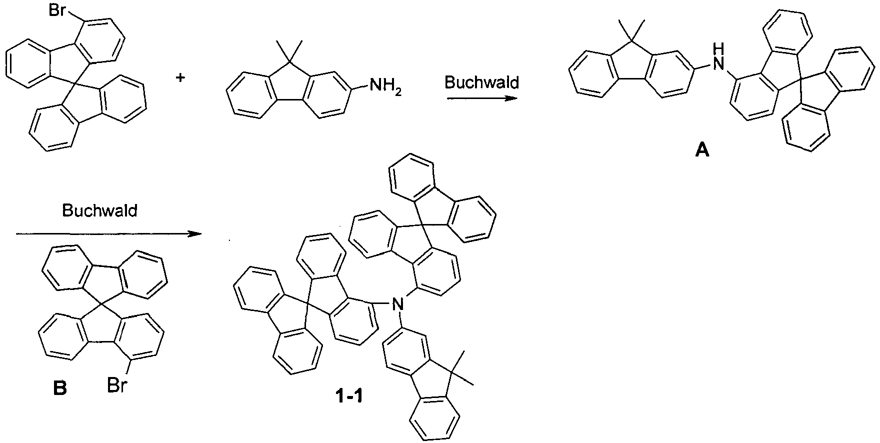

### Synthesis of (9,9-dimethyl-9H-fluoren-2-yl)-(9,9-spirobifluoren-4-yl)-amine A

1,1'-Bis(diphenylphosphino)ferrocene (0.83 g, 1.49 mmol), palladium acetate (0.34 mg, 1.49 mmol) and sodium tert-butoxide (9.3 g, 97.1 mmol) are added to a solution of fluoren-2-yl-amine (15.6 g, 97.1 mmol) and 4-bromo-9,9-spiro-bifluorene (30.0 g, 97.1 mmol) in degassed toluene (500 ml), and the mixture is heated under reflux for 20 h. The reaction mixture is cooled to room temperature, extended with toluene and filtered through Celite. The filtrate is extended with water, re-extracted with toluene, and the combined organic phases are dried and evaporated in vacuo. The residue is filtered through silica gel (heptane/dichloromethane) and crystallised from heptane/toluene. (9,9-dimethyl-9H-fluoren-2-yl)-(9,9-spirobifluoren-4-yl)-amine is obtained as a off-white solid (36.0 g, 92% of theory).

The following compounds **A-2** to **A-6** are obtained analogously:

| **Ex.** | **Bromo-Spiro** | **Aryl-amine** | **Product** | **Yield** |
|---|---|---|---|---|
| **A-1** | 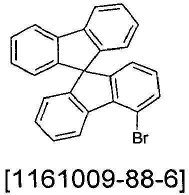 | 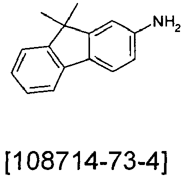 | 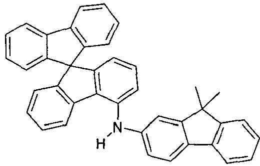 | 92% |
| **A-2** | 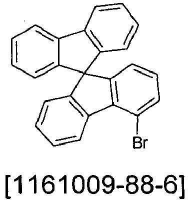 | 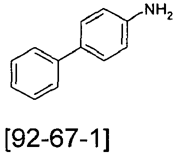 | 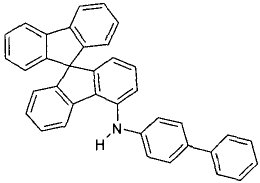 | 88% |
| **A-3** | 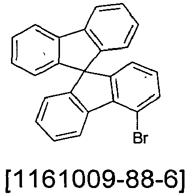 | 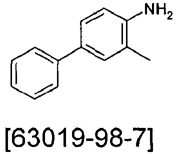 | 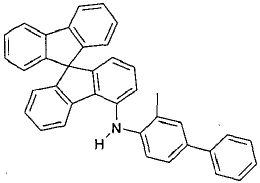 | 85% |
| **A-4** | 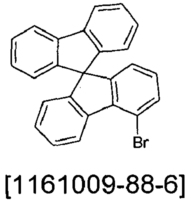 | 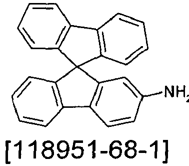 | 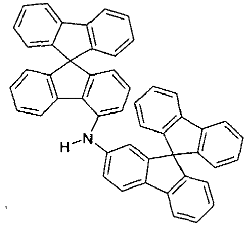 | 94% |
| **A-5** | 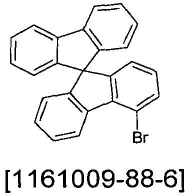 | 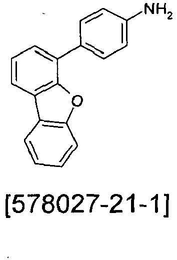 | 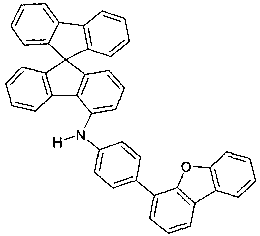 | 92% |
| **A-6** | 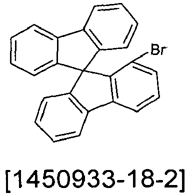 | 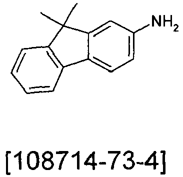 | 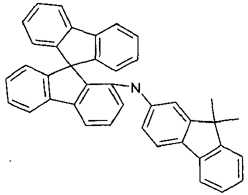 | 89% |
| **A-7** | 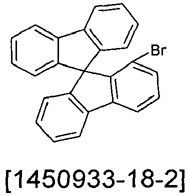 | 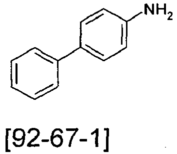 | 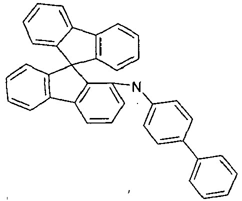 | 92% |
| **A-8** | 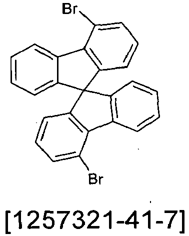 | 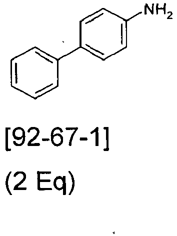 | 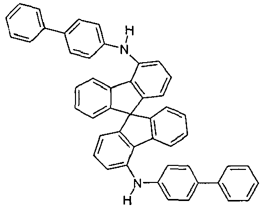 | 85% |

### Synthesis of Bis-(9,9-spirobifluoren-4-yl)-(9,9-dimethyl-9H-fluoren-2-yl)-amine (1-1)

Tri-tert-butylphosphine (1.69 ml of a 1.0 M solution in toluene, 1.69 mmol), palladium acetate (190 mg, 0.85 mmol) and sodium tert-butoxide (9.7 g, 101.4 mmol) are added to a solution of (9,9-dimethyl-9H-fluoren-2-yl)-(9,9-spirobifluoren-4-yl)-amine (18.0 g, 33.8 mmol) and 4-bromo-9,9'-spirobifluorene (13.6g, 33.8 mmol) in degassed toluene (400 ml), and the mixture is heated under reflux for 2 h. The reaction mixture is cooled to room temperature, extended with toluene and filtered through Celite. The filtrate is evaporated in vacuo, and the residue is crystallised from toluene/heptane. The crude product is extracted in a Soxhlet extractor (toluene) and purified by zone sublimation in vacuo twice. The product is isolated in the form of a white solid (19.8 g, 70% of theory, purity > 99.99% according to HPLC).

The following compounds are obtained analogously:

| **Ex.** | **Compound A** | **Compound B** | **Product** | **Yield** |
|---|---|---|---|---|
| **1-1** | 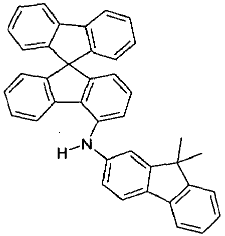 | 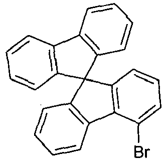 | 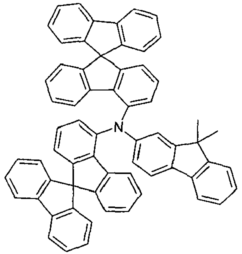 | 70% |
| **1-2** | 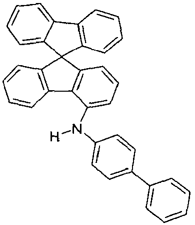 | 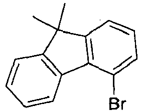 | 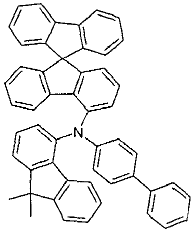 | 75% |
| **1-3** | 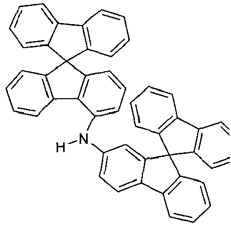 | 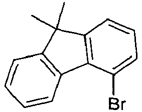 | 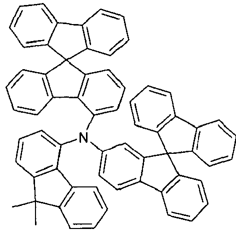 | 70% |
| **1-4** | 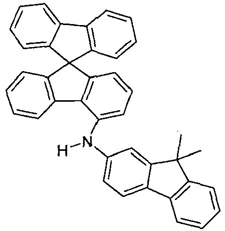 | 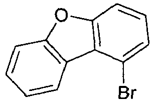 | 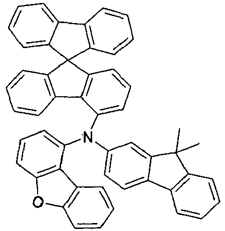 | 64% |
| **1-5** | 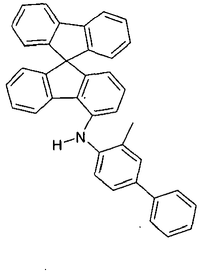 | 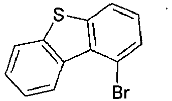 | 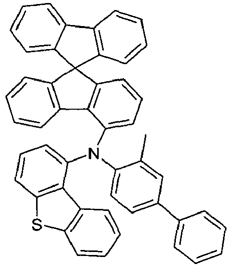 | 71% |
| **1-6** | 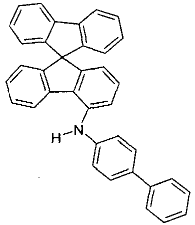 | 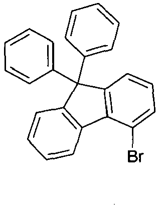 | 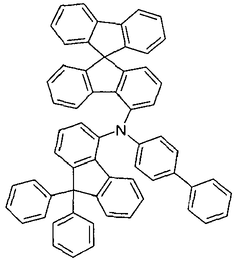 | 79% |
| **1-7** | 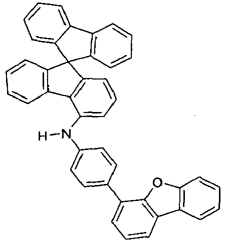 | 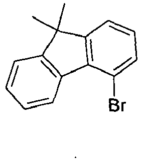 | 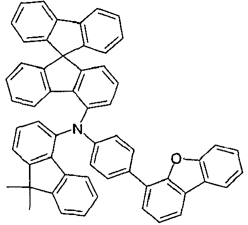 | 78% |
| **1-8** | 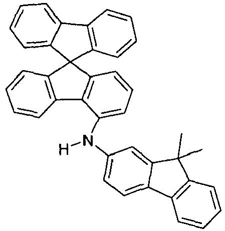 | 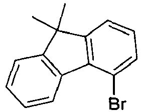 | 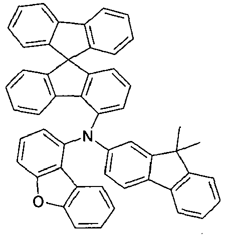 | 74% |
| **1-9** | 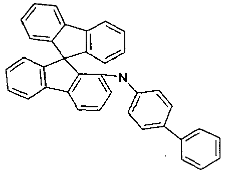 | 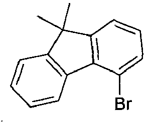 | 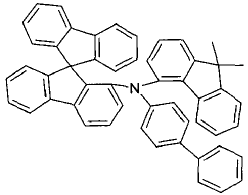 | 55% |
| **1-10** | 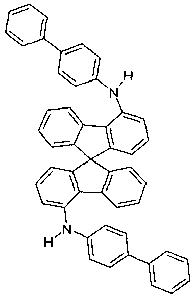 | 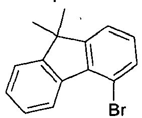 | 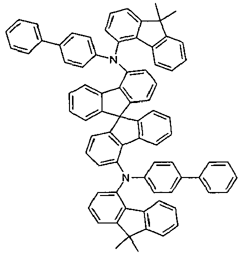 | 62% |

### B) Devices examples

OLEDs according to the invention and OLEDs in accordance with the prior art are produced by a general process in accordance with WO2004/058911, which is adapted to the circumstances described here (layer-thickness variation, materials).

The data for various OLEDs are presented in Examples E1 and E2 below (see Tables 1 to 4). The substrates used are glass plates coated with structured ITO (indium tin oxide) in a thickness of 50nm. The OLEDs basically have the following layer structure: substrate / hole-injection layer (HIL1)/hole-transport layer (HTL2) / hole-injection layer (HIL3) / electron-blocking layer (EBL) / emission layer (EML) / electron-transport layer (ETL) / electron-injection layer (EIL) and finally a cathode. The cathode is formed by an aluminium layer with a thickness of 100nm. The precise structure of the OLEDs is shown in Table 1 and 3. The materials required for the production of the OLEDs are shown in Table 5.

All materials are applied by thermal vapour deposition in a vacuum chamber. The emission layer here always consists of at least one matrix material (host material) and an emitting dopant (emitter), which is admixed with the matrix material or matrix materials in a certain proportion by volume by co-evaporation. An expression such as H1:SEB1(5%) here means that material H1 is present in the layer in a proportion by volume of 95% and SEB1 is present in the layer in a proportion of 5%. Analogously, the electron-transport layer may also consist of a mixture of two materials.

The OLEDs are characterised by standard methods. For this purpose, the electroluminescence spectra and the external quantum efficiency (EQE, measured in per cent) as a function of the luminous density, calculated from current/voltage/luminous density characteristic lines (IUL characteristic lines) assuming Lambert emission characteristics, and the lifetime are determined. The electroluminescence spectra are determined at a luminous density of 1000cd/m², and the CIE 1931 x and y colour coordinates are calculated therefrom. The expression EQE @ 1000cd/m² denotes the external quantum efficiency at an operating luminous density of 1000cd/m². LT80 @ 6000cd/m² is the lifetime until the OLED has dropped from a luminance of 6000cd/m² to 80% of the initial intensity, i.e. to 4800cd/m². The data for the various OLEDs are summarised in Tables 2 and 4.

### Use of compounds according to the invention as hole-transport materials in fluorescent OLEDs

In particular, compounds according to the invention are suitable as HIL, HTL, EBL or matrix material in the EML in OLEDs. They are suitable as a single layer, but also as mixed component as HIL, HTL, EBL or within the EML. Compared with components from prior art (V1,V2 and V3), the samples comprising the compounds according to the invention exhibit both higher efficiencies and also improved lifetimes both in singlet blue and also in triplet green.

| **Table 1: Structure of the OLEDs** | | | | | | |
|---|---|---|---|---|---|---|
| **Ex.** | **HIL** | **HTL** | **EBL** | **EML** | **ETL** | **EIL** |
| | Thickness / nm | Thickness / nm | Thickness / nm | Thickness / nm | Thickness / nm | Thickness / nm |
| E1 | HIM:F4TCNQ(5%) 20nm | HIM 170nm | HTM 20nm | H1 :SEB(5%) 20 nm | ETM1 :LiQ(50%) 30 nm | LiQ 1nm |
| V1 | HIM:F4TCNQ(5%) 20nm | HIM 170nm | HTMV1 20nm | H1 :SEB(5%) 20 nm | ETM1 :LiQ(50%) 30 nm | LiQ 1nm |
| V2 | HIM:F4TCNQ(5%) 20nm | HIM 170nm | HTMV2 20nm | H1 :SEB(5%) 20 nm | ETM1 :LiQ(50%) 30 nm | LiQ 1nm |
| V3 | HIM:F4TCNQ(5%) 20nm | HIM 170nm | HTMV3 20nm | H1:SEB(5%) 20 nm | ETM1 :LiQ(50%) 30 nm | LiQ 1nm |

| | **Table 2: Data for the OLEDs** | | |
|---|---|---|---|
| **Ex.** | **U @ 10mA/cm²** | **EQE @ 10mA/cm²** | **LT80 @ 60mA/cm²** |
| | [V] | % | [h] |
| E1 | 4.2 | 8.6 | 260 |
| V1 | 4.2 | 8.3 | 245 |
| V2 | 4.1 | 8.2 | 120 |
| V3 | 4.1 | 8.4 | 160 |

| **Table 3: Structure of the OLEDs** | | | | | | |
|---|---|---|---|---|---|---|
| **Ex.** | **HIL** | **HTL** | **EBL** | **EML** | **ETL** | **EIL** |
| | Thickness / nm | Thickness / nm | Thickness / nm | Thickness / nm | Thickness / nm | Thickness / nm |
| E2 | HIL2 70 nm | HIL1 5 nm | HIM 90 nm | ETM1:HTM1(42%):TEG(15%) 30 nm | ETM2(50%):LiQ(50%) 40 nm | --- |
| V4 | HIL2 70 nm | HIL1 5 nm | HIM 90 nm | ETM1 :HTMV1 (42%):TEG(15%) 30 nm | ETM2(50%):LiQ(50%) 40 nm | --- |
| V5 | HIL2 70 nm | HIL1 5 nm | HIM 90 nm | ETM1 :HTMV2(42%):TEG(15%) 30 nm | ETM2(50%):LiQ(50%) 40 nm | --- |
| V6 | HIL2 70 nm | HIL1 5 nm | HIM 90 nm | ETM1 :HTMV3(42%):TEG(15%) 30 nm | ETM2(50%):LiQ(50%) 40 nm | --- |

| **Table 4: Data for the OLEDs** | | | | |
|---|---|---|---|---|
| **Ex.** | **EQE @ 1000** cd/m² | **LT80 @ 20 mA/cm²** | **CIE** | |
| | | | **x** | **y** |
| E2 | 16.7% | 265 | 0.33 | 0.62 |
| V4 | 16.5% | 140 | 0.34 | 0.62 |
| V5 | 16.4% | 190 | 0.33 | 0.62 |
| V6 | 16.6% | 220 | 0.33 | 0.63 |

**Table 5 - Structures of the materials used**

| | | |
|---|---|---|
| 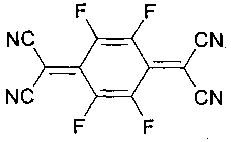 | 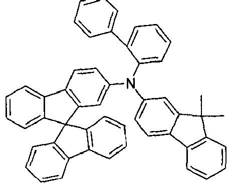 | 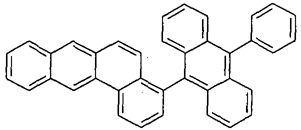 |
| F4TCNQ | HIM | H1 |
| 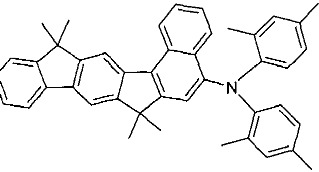 | 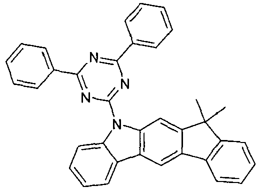 | 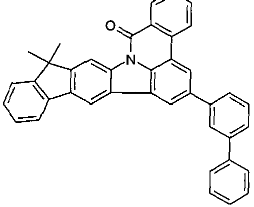 |
| SEB | H2 | ETM1 |
| 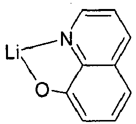 | 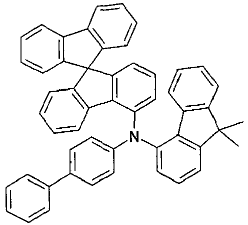 | 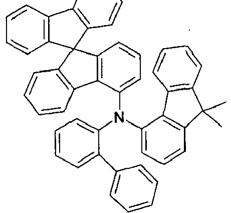 |
| LiQ | HTM1 | HTMV1 |
| 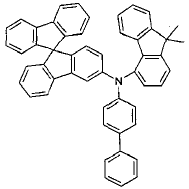 | 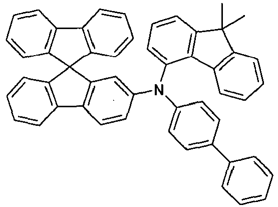 | 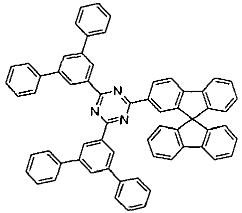 |
| HTMV2 | HTMV3 | ETM2 |
| 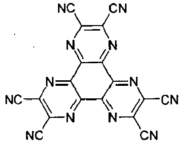 | 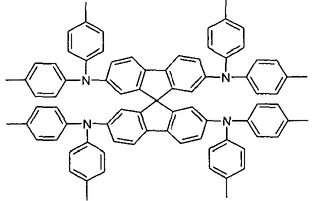 | 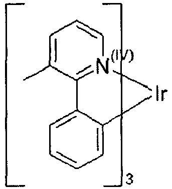 |
| HIL1 | HIL2 | TEG |

## Claims

1. Compound of the formula (1), 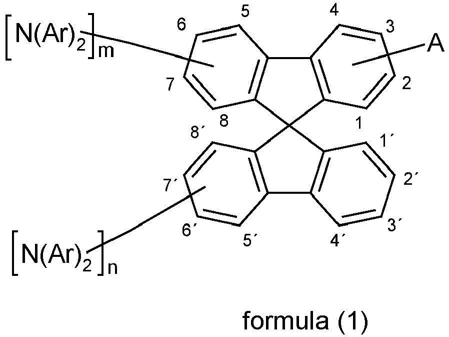 where A is a group of the following formula (2), which can only be present at the position 1 or position 4 of the spirobifluorene structure as depicted in formula (1) 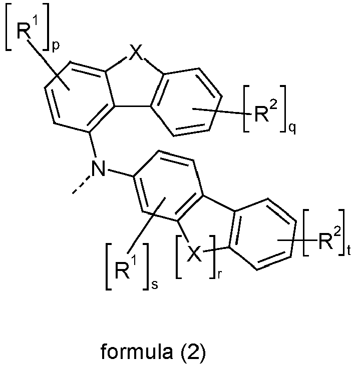
where the dashed bond indicates the bonding to the spirobifluorene structure;
where each free position 1 to 8 and 1 ' to 8' of the spirobifluorene structure, which is not substituted by a group A or N(Ar)₂, may be substituted by a group R;
and where the following applies to the symbols and indices used:
X is a divalent bridge selected from the group consisting of C(R⁰)₂, Si(R⁰)₂, C=O, O, S, S=O and SO₂; or X is a group of the following formula (3), 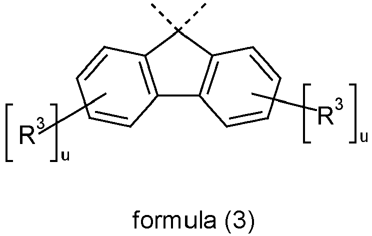 where the dashed bonds indicate the bonding to the fluorene group;
R, R⁰ are selected on each occurrence, identically or differently, from the group consisting of H, D, F, Cl, Br, I, CHO, CN, C(=O)Ar¹, P(=O)(Ar¹)₂, S(=O)Ar¹, S(=O)₂Ar¹, NO², Si(R⁴)₃, B(OR⁴)₂, OSO₂R⁴, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁴, where in each case one or more non-adjacent CH₂ groups may be replaced by R⁴C=CR⁴, C=C, Si(R⁴)₂, Ge(R⁴)₂, Sn(R⁴)₂, C=O, C=S, C=Se, P(=O)(R⁴), SO, SO₂, O, S or CONR⁴ and where one or more H atoms may be replaced by D, F, Cl, Br, I, CN or NO², an aromatic or heteroaromatic ring system having 5 to 40 aromatic ring atoms, which may in each case be substituted by one or more radicals R⁴, an aryloxy or heteroaryloxy group having 5 to 40 aromatic ring atoms, which may be substituted by one or more radicals R⁴, a thioaryl or thio-heteroaryl group having 5 to 40 aromatic ring atoms, which may be substituted by one or more radicals R⁴, where two substituents R⁰ attached to the same C or Si atom may optionally form a mono- or polycyclic aliphatic ring system, which may be substituted by one or more radicals R⁴;
R¹, R² and R³ are selected on each occurrence, identically or differently, from the group consisting of H, D, F, Cl, Br, I, CHO, CN, N(Ar¹)₂, C(=O)Ar¹, P(=O)(Ar¹)₂, S(=O)Ar¹, S(=O)₂Ar¹, NO², Si(R⁴)₃, B(OR⁴)₂, OSO₂R⁴, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁴, where in each case one or more non-adjacent CH₂ groups may be replaced by R⁴C=CR⁴, C≡C, Si(R⁴)₂, Ge(R⁴)₂, Sn(R⁴)₂, C=O, C=S, C=Se, P(=O)(R⁴), SO, SO₂, O, S or CONR⁴ and where one or more H atoms may be replaced by D, F, Cl, Br, I, CN or NO², an aromatic or heteroaromatic ring system having 5 to 40 aromatic ring atoms, which may in each case be substituted by one or more radicals R⁴, an aryloxy or heteroaryloxy group having 5 to 40 aromatic ring atoms, which may be substituted by one or more radicals R⁴, a thioaryl or thio-heteroaryl group having 5 to 40 aromatic ring atoms, which may be substituted by one or more radicals R⁴, where two or more adjacent substituents R¹, two or more adjacent substituents R² or two or more adjacent substituents R³, may optionally form a mono- or polycyclic, aliphatic ring system or aromatic ring system, which may be substituted by one or more radicals R⁴;
R⁴ is selected on each occurrence, identically or differently, from the group consisting of H, D, F, Cl, Br, I, CHO, CN, N(Ar¹)₂, C(=O)Ar¹, P(=O)(Ar¹)₂, S(=O)Ar¹, S(=O)₂Ar¹, NO², Si(R⁵)₃, B(OR⁵)₂, OSO₂R⁵, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁵, where in each case one or more non-adjacent CH₂ groups may be replaced by R⁵C=CR⁵, C=C, Si(R⁵)₂, Ge(R⁵)₂, Sn(R⁵)₂, C=O, C=S, C=Se, P(=O)(R⁵), SO, SO₂, O, S or CONR⁵ and where one or more H atoms may be replaced by D, F, Cl, Br, I, CN or NO², an aromatic or heteroaromatic ring system having 5 to 40 aromatic ring atoms, which may in each case be substituted by one or more radicals R⁵, an aryloxy or heteroaryloxy group having 5 to 40 aromatic ring atoms, which may be substituted by one or more radicals R⁵, a thioaryl or thio-heteroaryl group having 5 to 40 aromatic ring atoms, which may be substituted by one or more radicals R⁵, where two or more adjacent substituents R⁴ may optionally form a mono- or polycyclic, aliphatic ring system or aromatic ring system, which may be substituted by one or more radicals R⁵;
R⁵ is selected on each occurrence, identically or differently, from the group consisting of H, D, F, Cl, Br, I, CN, a straight-chain alkyl, alkoxy or thioalkyl group having 1 to 40 C atoms or a branched or cyclic alkyl, alkoxy or thioalkyl group having 3 to 40 C atoms, where in each case one or more non-adjacent CH₂ groups may be replaced by SO, SO₂, O, S and where one or more H atoms may be replaced by D, F, Cl, Br or I, an aromatic or heteroaromatic ring system having 5 to 60 aromatic ring atoms;
Ar, Ar¹ is on each occurrence, identically or differently, an aromatic or heteroaromatic ring system having 5 to 40 aromatic ring atoms, which may in each case also be substituted by one or more radicals R⁴;
m, n, r are, identically or differently, 0 or 1;
p is 3;
q, u are each 4;
s is 3 or 4, where s + r = 4;
t is 4 or 5, where t + r = 5.

2. Compound according to claim 1, **characterised in that** m + n = 0 or 1.

3. Compound according to claim 1 or 2, **characterised in that** X is selected from the group consisting of C(R⁰)₂, O, S, and SO₂ and r is equal to 0.

4. Compound according to claim one or more of the preceding claims, **characterised in that** X is selected from the group consisting of C(R⁰)₂ and r is equal to 0.

5. Compound according to claim one or more of the preceding claims, **characterised in that** the group N(Ar)² is a group A of formula (2) as defined in claim 1.

6. Compound according to one or more of the preceding claims, selected from the compounds of the formulae (1-1) to (1-6), 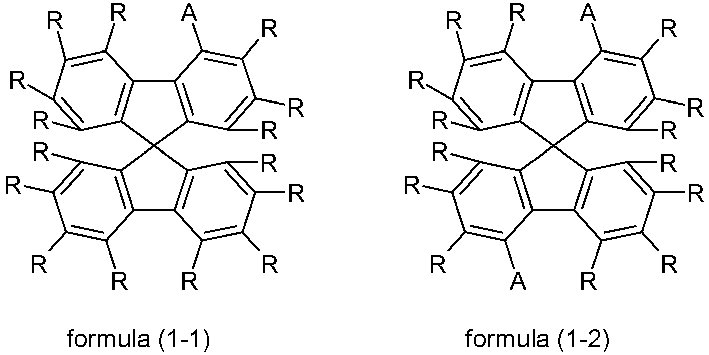 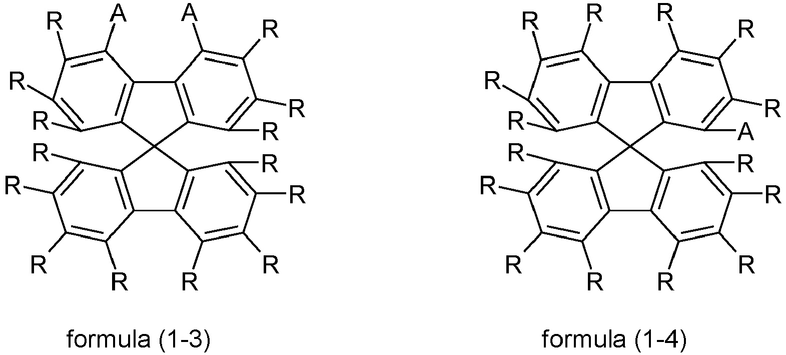 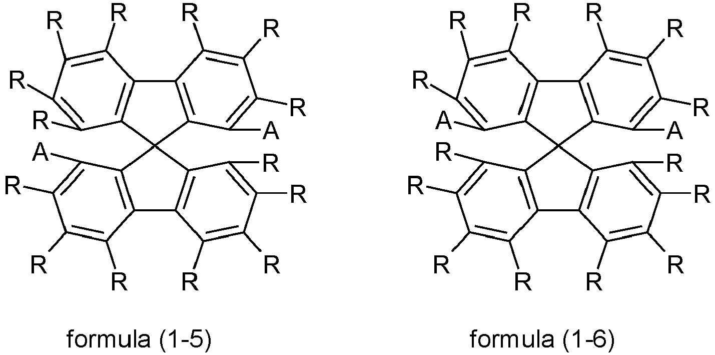 where the symbols and indices used have the meanings as given in claim 1.

7. Compound according to one or more of the preceding claims, selected from the compounds of the formulae (1-1 a) to (1-6a) 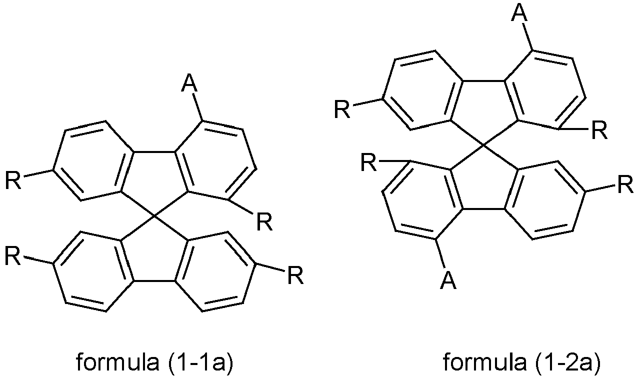 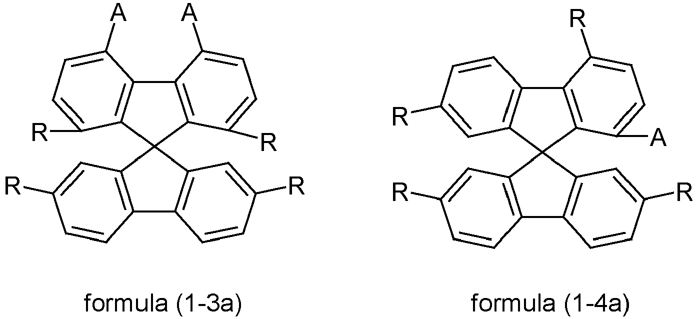 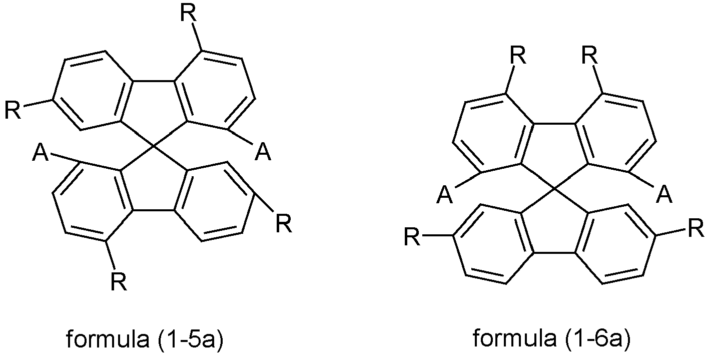 where the symbols and indices used have the meanings given in claim 1.

8. Compound according to one or more of the preceding claims, selected from the compounds of the formulae (1-1b) to (1-6b) 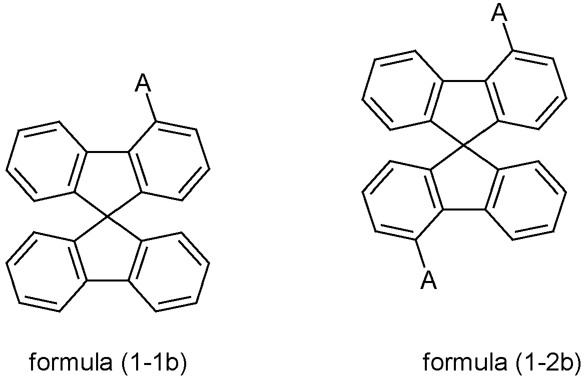 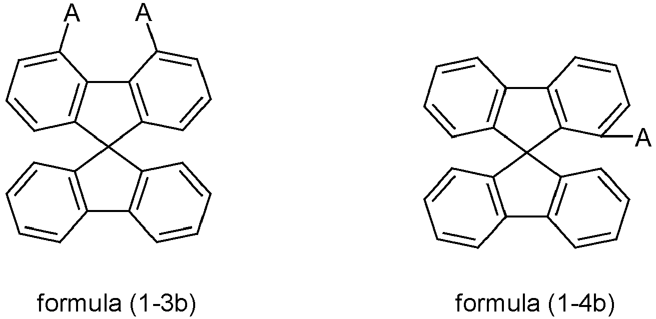 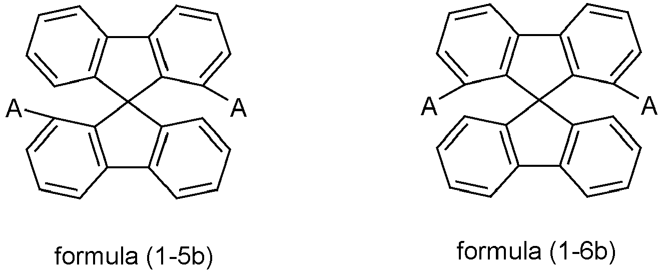 where the symbol A has the same meanings as in claim 1.

9. Compound according to one or more of the preceding claims, **characterised in that** R is selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R⁴.

10. Compound according to one or more of the preceding claims, **characterised in that** R² is selected on each occurrence, identically or differently, from the group consisting of H, D, F, CN, a straight-chain alkyl, or alkoxy group having 1 to 10 C atoms or a branched or cyclic alkyl or alkoxy group having 3 to 10 C atoms, each of which may be substituted by one or more radicals R⁴, where in each case one or more non-adjacent CH₂ groups may be replaced by O or S and where one or more H atoms may be replaced by F, an aryl or heteroaryl ring system having 5 to 24 aromatic ring atoms, which may in each case be substituted by one or more radicals R⁴, an aryloxy or heteroaryloxy group having 5 to 24 aromatic ring atoms, which may be substituted by one or more radicals R⁴, a thioaryl or thio-heteroaryl group having 5 to 24 aromatic ring atoms, which may be substituted by one or more radicals R⁴, where two or more adjacent substituents R², may optionally form a mono- or polycyclic, aliphatic ring system or aromatic ring system, which may be substituted by one or more radicals R⁴.

11. Compound according to one or more of the preceding claims, **characterised in that** R⁰ is selected on each occurrence, identically or differently, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where in each case one or more non-adjacent CH₂ groups may be replaced by O and where one or more H atoms may be replaced by D, F or CN, an aromatic or heteroaromatic ring system having 5 to 14 aromatic ring atoms, which may in each case be substituted by one or more radicals R⁴, where two substituents R⁰ may optionally form a monocyclic aliphatic ring system, which may be substituted by one or more radicals R⁴.

12. Compound according to one or more of the preceding claims, **characterised in that** R⁰ is selected on each occurrence, identically or differently, from the group consisting of H, a straight-chain alkyl group having 1 to 10 C atoms or a branched or cyclic alkyl group having 3 to 10 C atoms, each of which may be substituted by one or more radicals R⁴.

13. Process for the preparation of a compound according to one or more of the preceding claims, **characterised in that** the diarylamino groups A and N(Ar)² are introduced by a C-N coupling reaction between a 1- or 4-halogenated spirobifluorene and a diarylamine groups A or N(Ar)².

14. Formulation comprising at least one compound according to one or more of the claims 1 to 12 and at least one solvent.

15. Use of a compound according to one or more of claims 1 to 12 in an electronic device, in particular in an organic electroluminescent device.

16. Electronic device comprising at least one compound according to one or more of claims 1 to 12, preferably selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

17. Electronic device according to claim 16, which is an organic electroluminescent device, **characterised in that** the compound according to one or more of claims 1 to 12 is employed as hole-transport material in a hole-transport or hole-injection or exciton-blocking or electron-blocking layer or as matrix material for fluorescent or phosphorescent emitters.

## Patentansprüche

1. Verbindung der Formel (1), 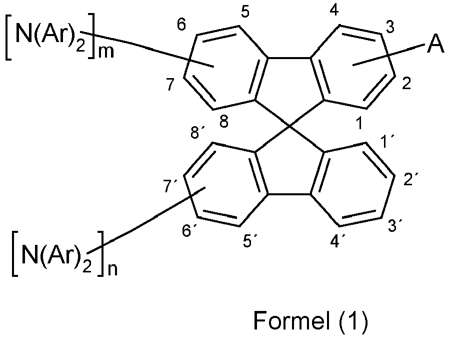 wobei A eine Gruppe der folgenden Formel (2) ist, die nur in Position 1 oder Position 4 der Spirobifluorenstruktur wie in Formel (1) dargestellt vorliegen kann 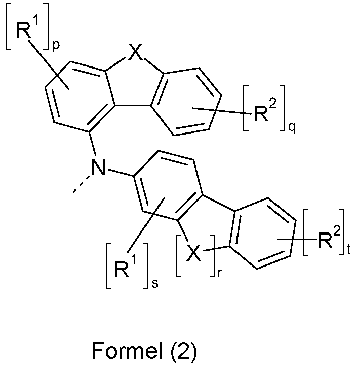
wobei die gestrichelte Bindung die Bindung an die Spirobifluorenstruktur angibt;
wobei jede freie Position 1 bis 8 und 1' bis 8' der Spirobifluorenstruktur, die nicht durch eine Gruppe A oder N(Ar)² substituiert ist, durch eine Gruppe R substituiert sein kann;
und wobei für die verwendeten Symbole und Indizes Folgendes gilt:
X ist eine zweiwertige Brücke, die aus der Gruppe bestehend aus C(R⁰)₂, Si(R⁰)₂, C=O, O, S, S=O und SO₂ ausgewählt ist; oder X ist eine Gruppe der folgenden Formel (3), 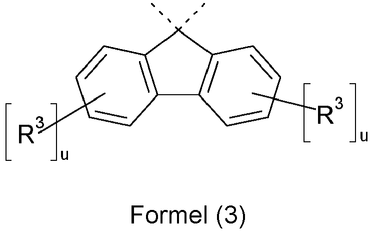 wobei die gestrichelten Bindungen die Bindung an die Fluorengruppe angeben;
R, R⁰ sind bei jedem Auftreten gleich oder verschieden ausgewählt aus der Gruppe bestehend aus H, D, F, Cl, Br, I, CHO, CN, C(=O)Ar¹, P(=O)(Ar¹)₂, S(=O)Ar¹, S(=O)₂Ar¹, NO₂, Si(R⁴)₃, B(OR⁴)₂, OSO₂R⁴, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils durch einen oder mehrere Reste R⁴ substituiert sein kann, wobei jeweils eine oder mehrere nicht benachbarte CH₂-Gruppen durch R⁴C=CR⁴, C≡C, Si(R⁴)₂, Ge(R⁴)₂, Sn(R⁴)₂, C=O, C=S, C=Se, P(=O)(R⁴), SO, SO₂, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils durch einen oder mehrere Reste R⁴ substituiert sein kann, einer Aryloxy- oder Heteroaryloxygruppe mit 5 bis 40 aromatischen Ringatomen, die durch einen oder mehrere Reste R⁴ substituiert sein kann, einer Thioaryl- oder Thio-heteroaryl-Gruppe mit 5 bis 40 aromatischen Ringatomen, die durch einen oder mehrere Reste R⁴ substituiert sein kann, wobei zwei Substituenten R⁰, die an dasselbe C-oder Si-Atom gebunden sind, gegebenenfalls ein mono- oder polycyclisches aliphatisches Ringsystem bilden können, das durch einen oder mehrere Reste R⁴ substituiert sein kann;
R¹, R² und R³ sind bei jedem Auftreten gleich oder verschieden ausgewählt aus der Gruppe bestehend aus H, D, F, Cl, Br, I, CHO, CN, N(Ar¹)₂, C(=O)Ar¹, P(=O)(Ar¹)₂, S(=O)Ar¹, S(=O)₂Ar¹, NO₂, Si(R⁴)₃, B(OR⁴)₂, OSO₂R⁴, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils durch einen oder mehrere Reste R⁴ substituiert sein kann, wobei jeweils eine oder mehrere nicht benachbarte CH₂-Gruppen durch R⁴C=CR⁴, C≡C, Si(R⁴)₂, Ge(R⁴)₂, Sn(R⁴)₂, C=O, C=S, C=Se, P(=O)(R⁴), SO, SO₂, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils durch einen oder mehrere Reste R⁴ substituiert sein kann, einer Aryloxy- oder Heteroaryloxygruppe mit 5 bis 40 aromatischen Ringatomen, die durch einen oder mehrere Reste R⁴ substituiert sein kann, einer Thioaryl- oder Thio-heteroaryl-Gruppe mit 5 bis 40 aromatischen Ringatomen, die durch einen oder mehrere Reste R⁴ substituiert sein kann, wobei zwei oder mehr benachbarte Substituenten R¹, zwei oder mehr benachbarte Substituenten R² oder zwei oder mehr benachbarte Substituenten R³ gegebenenfalls ein mono- oder polycyclisches, aliphatisches Ringsystem oder aromatisches Ringsystem bilden können, das durch einen oder mehrere Reste R⁴ substituiert sein kann;
R⁴ ist bei jedem Auftreten gleich oder verschieden ausgewählt aus der Gruppe bestehend aus H, D, F, Cl, Br, I, CHO, CN, N(Ar¹)₂, C(=O)Ar¹, P(=O)(Ar¹)₂, S(=O)Ar¹, S(=O)₂Ar¹, NO₂, Si(R⁵)₃, B(OR⁵)₂, OSO₂R⁵, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils durch einen oder mehrere Reste R⁵ substituiert sein kann, wobei jeweils eine oder mehrere nicht benachbarte CH₂-Gruppen durch R⁵C=CR⁵, C≡C, Si(R⁵)₂, Ge(R⁵)₂, Sn(R⁵)₂, C=O, C=S, C=Se, P(=O)(R⁵), SO, SO₂, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils durch einen oder mehrere Reste R⁵ substituiert sein kann, einer Aryloxy- oder Heteroaryloxygruppe mit 5 bis 40 aromatischen Ringatomen, die durch einen oder mehrere Reste R⁵ substituiert sein kann, einer Thioaryl- oder Thio-heteroaryl-Gruppe mit 5 bis 40 aromatischen Ringatomen, die durch einen oder mehrere Reste R⁵ substituiert sein kann, wobei zwei oder mehr benachbarte Substituenten R⁴ gegebenenfalls ein mono- oder polycyclisches, aliphatisches Ringsystem oder aromatisches Ringsystem bilden können, das durch einen oder mehrere Reste R⁵ substituiert sein kann;
R⁵ ist bei jedem Auftreten gleich oder verschieden ausgewählt aus der Gruppe bestehend aus H, D, F, Cl, Br, I, CN, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, wobei jeweils eine oder mehrere nicht benachbarte CH₂-Gruppen durch SO, SO₂, O, S ersetzt sein können und wobei ein oder mehrere H-Atome durch D, F, Cl, Br oder I ersetzt sein können, einem aromatischen oder heteroaromatischen Ringsystem mit 5 bis 60 aromatischen Ringatomen;
Ar, Ar¹ ist bei jedem Auftreten gleich oder verschieden ein aromatisches oder heteroaromatisches Ringsystem mit 5 bis 40 aromatischen Ringatomen, das jeweils auch durch einen oder mehrere Reste R⁴ substituiert sein kann;
m, n, r sind gleich oder verschieden 0 oder 1;
p ist 3;
q, u sind jeweils 4;
s ist 3 oder 4, wobei s + r = 4;
t ist 4 oder 5, wobei t + r = 5.

2. Verbindung nach Anspruch 1, **dadurch gekennzeichnet, dass** m + n = 0 oder 1.

3. Verbindung nach Anspruch 1 oder 2, **dadurch gekennzeichnet, dass** X aus der Gruppe bestehend aus C(R⁰)₂, O, S und SO₂ ausgewählt ist und r gleich 0 ist.

4. Verbindung nach einem oder mehreren der vorhergehenden Ansprüche, **dadurch gekennzeichnet, dass** X aus der Gruppe bestehend aus C(R⁰)₂ ausgewählt ist und r gleich 0 ist.

5. Verbindung nach einem oder mehreren der vorhergehenden Ansprüche, **dadurch gekennzeichnet, dass** die Gruppe N(Ar)² eine Gruppe A der Formel (2) wie in Anspruch 1 definiert ist.

6. Verbindung nach einem oder mehreren der vorhergehenden Ansprüche, ausgewählt aus den Verbindungen der Formeln (1-1) bis (1-6), 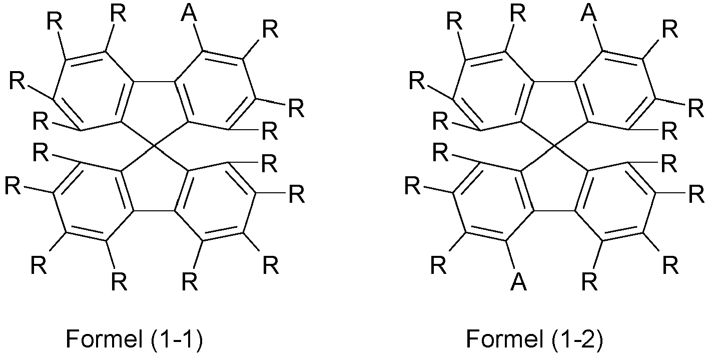 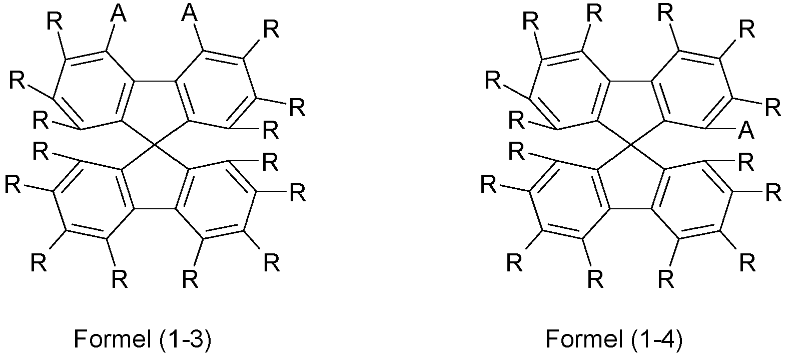 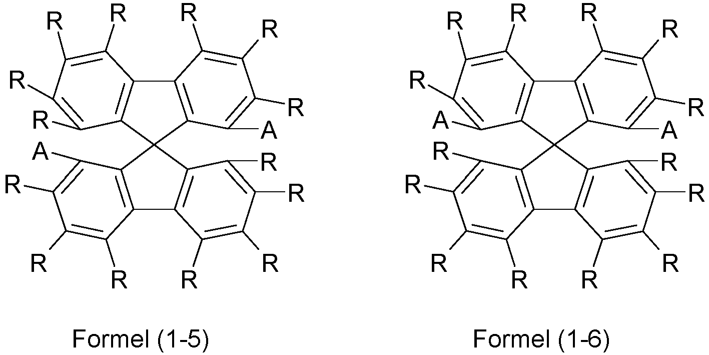 wobei die verwendeten Symbole und Indizes die wie in Anspruch 1 gegebenen Bedeutungen besitzen.

7. Verbindung nach einem oder mehreren der vorhergehenden Ansprüche, ausgewählt aus den Verbindungen der Formeln (1-1a) bis (1-6a) 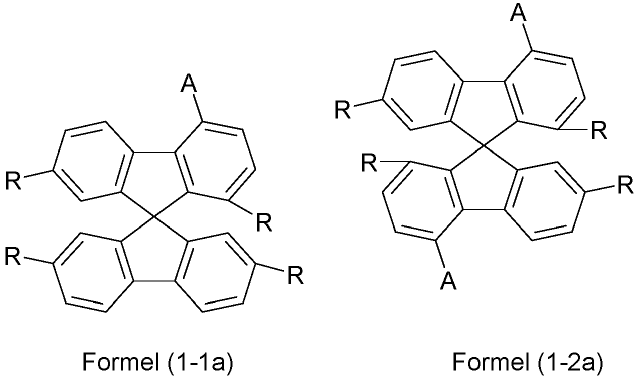 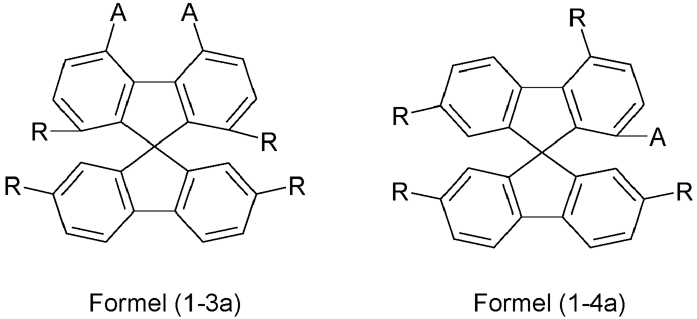 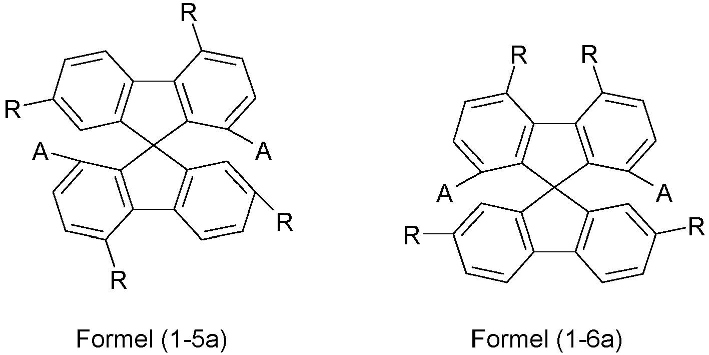 wobei die verwendeten Symbole und Indizes die in Anspruch 1 gegebenen Bedeutungen besitzen.

8. Verbindung nach einem oder mehreren der vorhergehenden Ansprüche, ausgewählt aus den Verbindungen der Formeln (1-1b) bis (1- 6b) 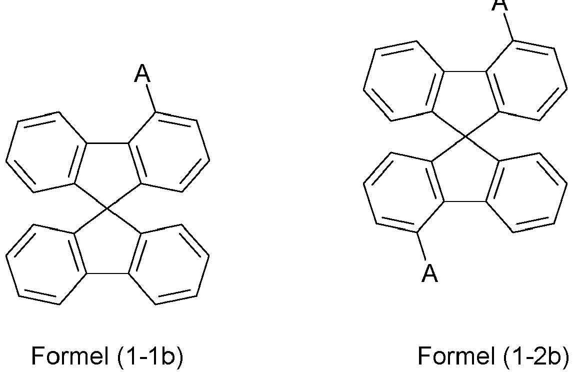 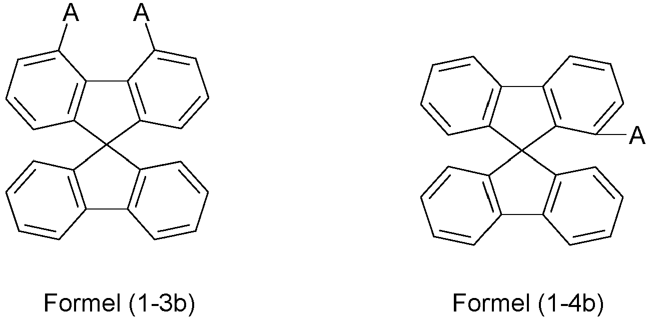 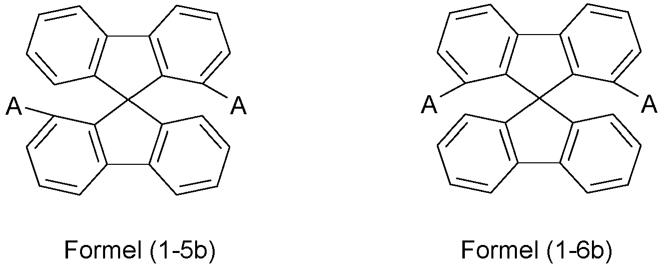 wobei das Symbol A die gleichen Bedeutungen wie in Anspruch 1 besitzt.

9. Verbindung nach einem oder mehreren der vorhergehenden Ansprüche, **dadurch gekennzeichnet, dass** R gleich oder verschieden bei jedem Auftreten ausgewählt ist aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils durch einen oder mehrere Reste R⁴ substituiert sein kann, wobei eine oder mehrere nicht benachbarte CH₂-Gruppen durch O ersetzt sein können und wobei ein oder mehrere H-Atome durch F ersetzt sein können, einem aromatischen oder heteroaromatischen Ringsystem mit 5 bis 24 aromatischen Ringatomen, das jeweils durch einen oder mehrere Reste R⁴ substituiert sein kann.

10. Verbindung nach einem oder mehreren der vorhergehenden Ansprüche, **dadurch gekennzeichnet, dass** R² bei jedem Auftreten gleich oder verschieden ausgewählt ist aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils durch einen oder mehrere Reste R⁴ substituiert sein kann, wobei jeweils eine oder mehrere nicht benachbarte CH₂-Gruppen durch O oder S ersetzt sein können und wobei ein oder mehrere H-Atome durch F ersetzt sein können, einem Aryl- oder Heteroaryl-Ringsystem mit 5 bis 24 aromatischen Ringatomen, das jeweils durch einen oder mehrere Reste R⁴ substituiert sein kann, einer Aryloxy- oder Heteroaryloxygruppe mit 5 bis 24 aromatischen Ringatomen, die durch einen oder mehrere Reste R⁴ substituiert sein kann, einer Thioaryl- oder Thio-heteroaryl-Gruppe mit 5 bis 24 aromatischen Ringatomen, die durch einen oder mehrere Reste R⁴ substituiert sein kann, wobei zwei oder mehr benachbarte Substituenten R² gegebenenfalls ein mono- oder polycyclisches aliphatisches Ringsystem oder aromatisches Ringsystem bilden können, das durch einen oder mehrere Reste R⁴ substituiert sein kann.

11. Verbindung nach einem oder mehreren der vorhergehenden Ansprüche, **dadurch gekennzeichnet, dass** R⁰ bei jedem Auftreten gleich oder verschieden ausgewählt ist aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils durch einen oder mehrere Reste R⁴ substituiert sein kann, wobei jeweils eine oder mehrere nicht benachbarte CH₂-Gruppen durch O ersetzt sein können und wobei ein oder mehrere H-Atome durch D, F oder CN ersetzt sein können, einem aromatischen oder heteroaromatischen Ringsystem mit 5 bis 14 aromatischen Ringatomen, das jeweils durch einen oder mehrere Reste R⁴ substituiert sein kann, wobei zwei Substituenten R⁰ gegebenenfalls ein monocyclisches aliphatisches Ringsystem bilden können, das durch einen oder mehrere Reste R⁴ substituiert sein kann.

12. Verbindung nach einem oder mehreren der vorhergehenden Ansprüche, **dadurch gekennzeichnet, dass** R⁰ bei jedem Auftreten gleich oder verschieden ausgewählt ist aus der Gruppe bestehend aus H, einer geradkettigen Alkylgruppe mit 1 bis 10 C-Atomen oder einer verzweigten oder cyclischen Alkylgruppe mit 3 bis 10 C-Atomen, die jeweils durch einen oder mehrere Reste R⁴ substituiert sein kann.

13. Verfahren zur Herstellung einer Verbindung nach einem oder mehreren der vorhergehenden Ansprüche, **dadurch gekennzeichnet, dass** die Diarylaminogruppen A und N(Ar)² durch eine C-N-Kupplungsreaktion zwischen einem 1- oder 4-halogenierten Spirobifluoren und einer Diarylamingruppe A oder N(Ar)² eingeführt werden.

14. Formulierung enthaltend mindestens eine Verbindung nach einem oder mehreren der Ansprüche 1 bis 12 sowie mindestens ein Lösungsmittel.

15. Verwendung einer Verbindung nach einem oder mehreren der Ansprüche 1 bis 12 in einer elektronischen Vorrichtung, insbesondere in einer organischen Elektrolumineszenzvorrichtung.

16. Elektronische Vorrichtung enthaltend mindestens eine Verbindung nach einem oder mehreren der Ansprüche 1 bis 12, vorzugsweise ausgewählt aus der Gruppe bestehend aus organischen Elektrolumineszenzvorrichtungen, organischen integrierten Schaltungen, organischen Feldeffekttransistoren, organischen Dünnschichttransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und organischen Plasmon-emittierenden Vorrichtungen.

17. Elektronische Vorrichtung nach Anspruch 16, bei der es sich um eine organische Elektrolumineszenzvorrichtung handelt, **dadurch gekennzeichnet, dass** die Verbindung nach einem oder mehreren der Ansprüche 1 bis 12 als Lochtransportmaterial in einer Lochtransport- oder Lochinjektions- oder Exzitonenblockier- oder Elektronenblockierschicht oder als Matrixmaterial für fluoreszierende oder phosphoreszierende Emitter eingesetzt wird.

## Revendications

1. Composé de la formule (1), 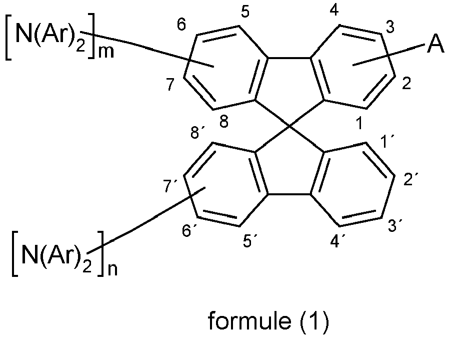 dans laquelle A est un groupe de la formule qui suit (2), lequel peut seulement être présent au niveau de la position 1 ou de la position 4 de la structure spirobifluorène telle que représentée selon la formule (1) 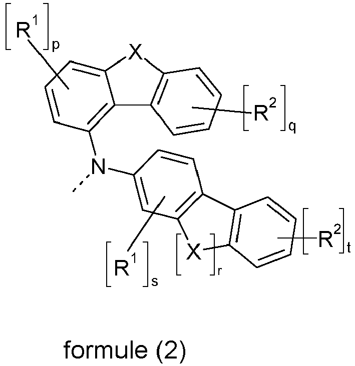
dans laquelle la liaison en pointillés indique la liaison sur la structure spirobifluorène ;
dans laquelle chacune des positions libres 1 à 8 et 1' à 8' de la structure spirobifluorène, qui n'est pas substituée par un groupe A ou par N(Ar)₂, peut être substituée par un groupe R ;
et dans laquelle ce qui suit s'applique aux symboles et aux indices qui sont utilisés :
X est un pont divalent qui est sélectionné parmi le groupe qui est constitué par C(R⁰)₂, Si(R⁰)₂, C=O, O, S, S=O et SO₂;
ou X est un groupe de la formule qui suit (3), 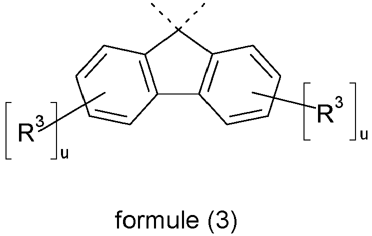 dans laquelle les liaisons en pointillés indiquent la liaison sur le groupe fluorène ;
R, R⁰ sont sélectionnés pour chaque occurrence, de manière identique ou différente, parmi le groupe qui est constitué par H, D, F, Cl, Br, I, CHO, CN, C(=O)Ar¹, P(=O)(Ar¹)₂, S(=O)Ar¹, S(=O)₂Ar¹, NO₂, Si(R⁴)₃, B(OR⁴)₂, OSO₂R⁴, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C, dont chacun peut être substitué par un radical ou par plusieurs radicaux R⁴, où, dans chaque cas, un ou plusieurs groupe(s) CH₂ non adjacents peut/peuvent être remplacé(s) par R⁴C=CR⁴, C≡C, Si(R⁴)₂, Ge(R⁴)₂, Sn(R⁴)₂, C=O, C=S, C=Se, P(=O)(R⁴), SO, SO₂, O, S ou CONR⁴ et où un ou plusieurs atome(s) de H peut/peuvent être remplacé(s) par D, F, Cl, Br, I, CN ou NO₂, un système de cycle aromatique ou hétéroaromatique qui comporte de 5 à 40 atomes de cycle aromatique, lequel peut dans chaque cas être substitué par un radical ou par plusieurs radicaux R⁴, un groupe aryloxy ou hétéroaryloxy qui comporte de 5 à 40 atomes de cycle aromatique, lequel peut être substitué par un radical ou par plusieurs radicaux R⁴, un groupe thioaryle ou thio-hétéroaryle qui comporte de 5 à 40 atomes de cycle aromatique, lequel peut être substitué par un radical ou par plusieurs radicaux R⁴, où deux substituants R⁰ qui sont liés au même atome de C ou de Si peuvent en option former un système de cycle aliphatique mono- ou polycyclique, lequel peut être substitué par un radical ou par plusieurs radicaux R⁴ ;
R¹, R² et R³ sont sélectionnés pour chaque occurrence, de manière identique ou différente, parmi le groupe qui est constitué par H, D, F, Cl, Br, I, CHO, CN, N(Ar¹)₂, C(=O)Ar¹, P(=O)(Ar¹)₂, S(=O)Ar¹, S(=O)₂Ar¹, NO₂, Si(R⁴)₃, B(OR⁴)₂, OSO₂R⁴, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C, dont chacun peut être substitué par un radical ou par plusieurs radicaux R⁴, où, dans chaque cas, un ou plusieurs groupe(s) CH₂ non adjacents peut/peuvent être remplacé(s) par R⁴C=CR⁴, C=C, Si(R⁴)₂, Ge(R⁴)₂, Sn(R⁴)₂, C=O, C=S, C=Se, P(=O)(R⁴), SO, SO₂, O, S ou CONR⁴ et où un ou plusieurs atome(s) de H peut/peuvent être remplacé(s) par D, F, Cl, Br, I, CN ou NO₂, un système de cycle aromatique ou hétéroaromatique qui comporte de 5 à 40 atomes de cycle aromatique, lequel peut dans chaque cas être substitué par un radical ou par plusieurs radicaux R⁴, un groupe aryloxy ou hétéroaryloxy qui comporte de 5 à 40 atomes de cycle aromatique, lequel peut être substitué par un radical ou par plusieurs radicaux R⁴, un groupe thioaryle ou thio-hétéroaryle qui comporte de 5 à 40 atomes de cycle aromatique, lequel peut être substitué par un radical ou par plusieurs radicaux R⁴, où deux substituants adjacents R¹ ou plus, deux substituants adjacents R² ou plus ou deux substituants adjacents R³ ou plus peuvent en option former un système de cycle aliphatique ou un système de cycle aromatique mono- ou polycyclique, lequel peut être substitué par un radical ou par plusieurs radicaux R⁴ ;
R⁴ est sélectionné pour chaque occurrence, de manière identique ou différente, parmi le groupe qui est constitué par H, D, F, Cl, Br, I, CHO, CN, N(Ar¹)₂, C(=O)Ar¹, P(=O)(Ar¹)₂, S(=O)Ar¹, S(=O)₂Ar¹, NO₂, Si(R⁵)₃, B(OR⁵)₂, OSO₂R⁵, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C, dont chacun peut être substitué par un radical ou par plusieurs radicaux R⁵, où, dans chaque cas, un ou plusieurs groupe(s) CH₂ non adjacents peut/peuvent être remplacé(s) par R⁵C=CR⁵, C≡C, Si(R⁵)₂, Ge(R⁵)₂, Sn(R⁵)₂, C=O, C=S, C=Se, P(=O)(R⁵), SO, SO₂, O, S ou CONR⁵ et où un ou plusieurs atome(s) de H peut/peuvent être remplacé(s) par D, F, Cl, Br, I, CN ou NO₂, un système de cycle aromatique ou hétéroaromatique qui comporte de 5 à 40 atomes de cycle aromatique, lequel peut dans chaque cas être substitué par un radical ou par plusieurs radicaux R⁵, un groupe aryloxy ou hétéroaryloxy qui comporte de 5 à 40 atomes de cycle aromatique, lequel peut être substitué par un radical ou par plusieurs radicaux R⁵, un groupe thioaryle ou thio-hétéroaryle qui comporte de 5 à 40 atomes de cycle aromatique, lequel peut être substitué par un radical ou par plusieurs radicaux R⁵, où deux substituants adjacents R⁴ ou plus peuvent en option former un système de cycle aliphatique ou un système de cycle aromatique mono- ou polycyclique, lequel peut être substitué par un radical ou par plusieurs radicaux R⁵ ;
R⁵ est sélectionné pour chaque occurrence, de manière identique ou différente, parmi le groupe qui est constitué par H, D, F, Cl, Br, I, CN, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C, où, dans chaque cas, un ou plusieurs groupe(s) CH₂ non adjacents peut/ peuvent être remplacé(s) par SO, SO₂, O, S et où un ou plusieurs atome(s) de H peut/peuvent être remplacé(s) par D, F, Cl, Br ou I, un système de cycle aromatique ou hétéroaromatique qui comporte de 5 à 60 atomes de cycle aromatique ;
Ar, Ar¹ sont pour chaque occurrence, de manière identique ou différente, un système de cycle aromatique ou hétéroaromatique qui comporte de 5 à 40 atomes de cycle aromatique, lequel peut dans chaque cas être substitué par un radical ou par plusieurs radicaux R⁴ ;
m, n, r sont, de manière identique ou différente, 0 ou 1 ;
p est 3 ;
q, u sont chacun 4 ;
s est 3 ou 4, où s + r = 4 ;
t est 4 ou 5, où t + r = 5.

2. Composé selon la revendication 1, **caractérisé en ce que** m + n = 0 ou 1.

3. Composé selon la revendication 1 ou 2, **caractérisé en ce que** X est sélectionné parmi le groupe qui est constitué par C(R⁰)₂, O, S, et SO₂ et r est égal à 0.

4. Composé selon une ou plusieurs des revendications qui précèdent, **caractérisé en ce que** X est sélectionné parmi le groupe qui est constitué par C(R⁰)₂ et r est égal à 0.

5. Composé selon une ou plusieurs des revendications qui précèdent, **caractérisé en ce que** le groupe N(Ar)² est un groupe A de la formule (2) tel qu'il a été défini selon la revendication 1.

6. Composé selon une ou plusieurs des revendications qui précèdent, sélectionné parmi les composés des formules (1-1) à (1-6), 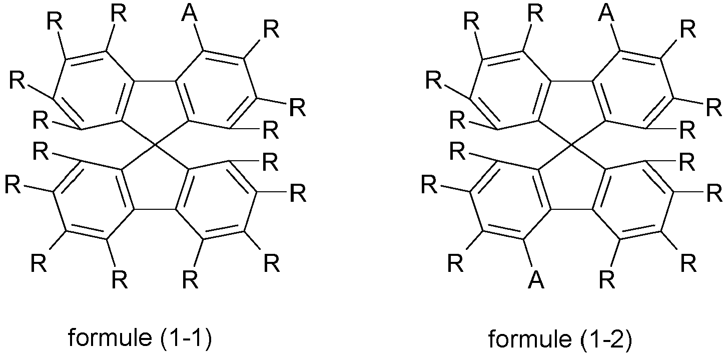 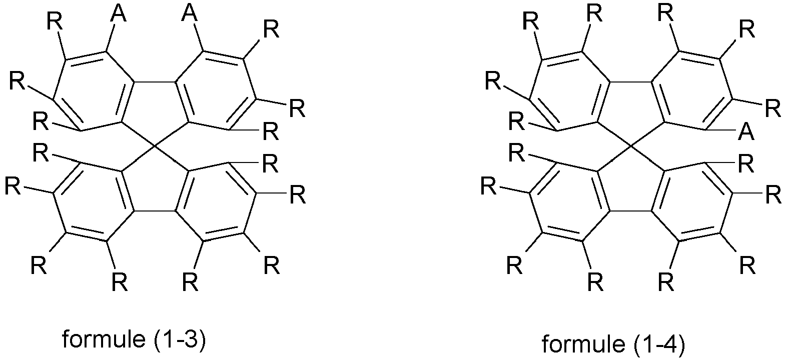 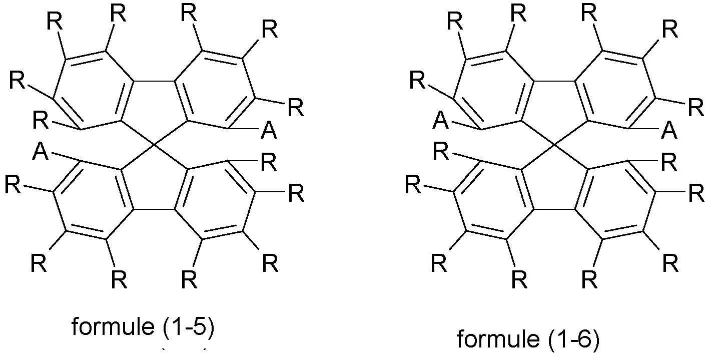 dans lesquelles les symboles et les indices qui sont utilisés présentent les significations qui ont été données selon la revendication 1.

7. Composé selon une ou plusieurs des revendications qui précèdent, sélectionné parmi les composés des formules (1-1a) à (1-6a) 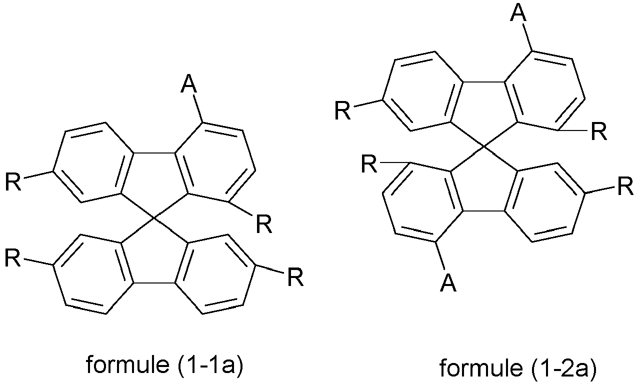 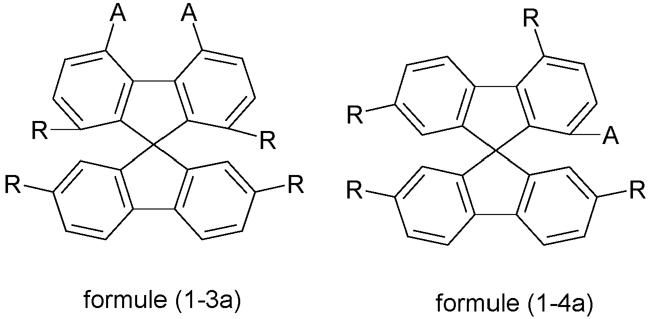 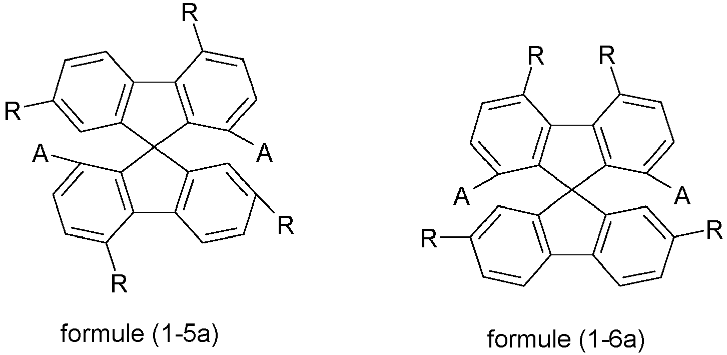 dans lesquelles les symboles et les indices qui sont utilisés présentent les significations qui ont été données selon la revendication 1.

8. Composé selon une ou plusieurs des revendications qui précèdent, sélectionné parmi les composés des formules (1-1b) à (1-6b) 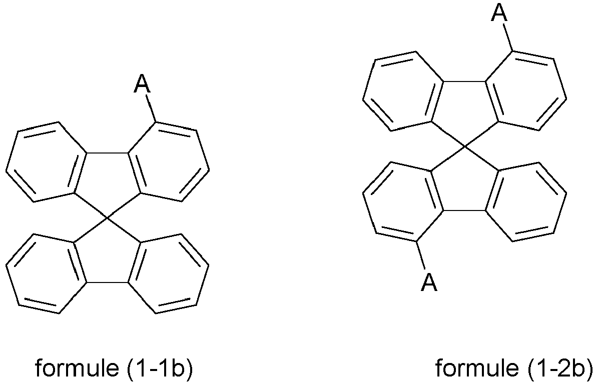 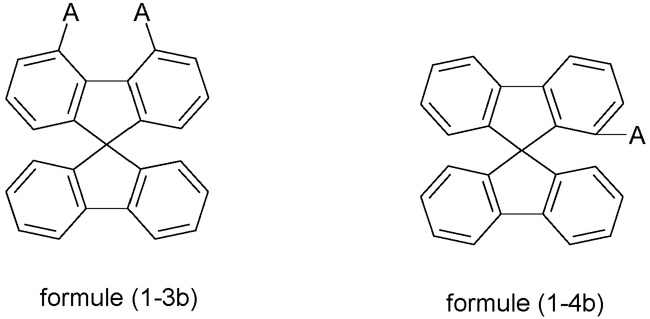 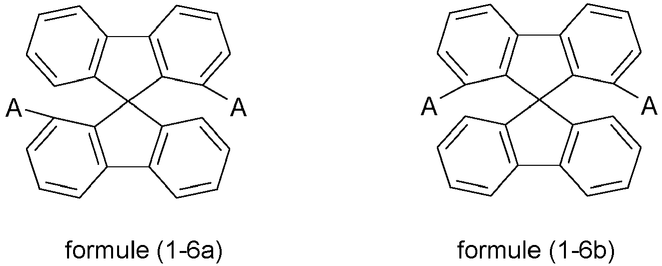 dans lesquelles le symbole A présente les mêmes significations que selon la revendication 1.

9. Composé selon une ou plusieurs des revendications qui précèdent, **caractérisé en ce que** R est sélectionné, de manière identique ou différente pour chaque occurrence, parmi le groupe qui est constitué par H, D, F, CN, un groupe alkyle ou alcoxy en chaîne droite qui comporte de 1 à 10 atome(s) de C ou un groupe alkyle ou alcoxy ramifié ou cyclique qui comporte de 3 à 10 atomes de C, dont chacun peut être substitué par un radical ou par plusieurs radicaux R⁴, où un ou plusieurs groupe(s) CH₂ non adjacents peut/peuvent être remplacé(s) par O et où un ou plusieurs atomes de H peut/peuvent être remplacé(s) par F, un système de cycle aromatique ou hétéroaromatique qui comporte de 5 à 24 atomes de cycle aromatique, lequel peut dans chaque cas être substitué par un radical ou par plusieurs radicaux R⁴.

10. Composé selon une ou plusieurs des revendications qui précèdent, **caractérisé en ce que** R² est sélectionné pour chaque occurrence, de manière identique ou différente, parmi le groupe qui est constitué par H, D, F, CN, un groupe alkyle ou alcoxy en chaîne droite qui comporte de 1 à 10 atome(s) de C ou un groupe alkyle ou alcoxy ramifié ou cyclique qui comporte de 3 à 10 atomes de C, dont chacun peut être substitué par un radical ou par plusieurs radicaux R⁴, où, dans chaque cas, un ou plusieurs groupe(s) CH₂ non adjacents peut/ peuvent être remplacé(s) par O ou par S et où un ou plusieurs atome(s) de H peut/peuvent être remplacé(s) par F, un système de cycle aryle ou hétéroaryle qui comporte de 5 à 24 atomes de cycle aromatique, lequel peut dans chaque cas être substitué par un radical ou par plusieurs radicaux R⁴, un groupe aryloxy ou hétéroaryloxy qui comporte de 5 à 24 atomes de cycle aromatique, lequel peut être substitué par un radical ou par plusieurs radicaux R⁴, un groupe thioaryle ou thio-hétéroaryle qui comporte de 5 à 24 atomes de cycle aromatique, lequel peut être substitué par un radical ou par plusieurs radicaux R⁴, où deux substituants R² adjacents ou plus peuvent en option former un système de cycle aliphatique ou un système de cycle aromatique mono- ou polycyclique, lequel peut être substitué par un radical ou par plusieurs radicaux R⁴.

11. Composé selon une ou plusieurs des revendications qui précèdent, **caractérisé en ce que** R⁰ est sélectionné pour chaque occurrence, de manière identique ou différente, parmi le groupe qui est constitué par H, D, F, CN, un groupe alkyle ou alcoxy en chaîne droite qui comporte de 1 à 10 atome(s) de C ou un groupe alkyle ou alcoxy ramifié ou cyclique qui comporte de 3 à 10 atomes de C, dont chacun peut être substitué par un radical ou par plusieurs radicaux R⁴, où dans chaque cas, un ou plusieurs groupe(s) CH₂ non adjacents peut/ peuvent être remplacé(s) par O et où un ou plusieurs atome(s) de H peut/peuvent être remplacé(s) par D, F ou CN, un système de cycle aromatique ou hétéroaromatique qui comporte de 5 à 14 atomes de cycle aromatique, lequel peut dans chaque cas être substitué par un radical ou par plusieurs radicaux R⁴, où deux substituants R⁰ peuvent en option former un système de cycle aliphatique monocyclique, lequel peut être substitué par un radical ou par plusieurs radicaux R⁴.

12. Composé selon une ou plusieurs des revendications qui précèdent, **caractérisé en ce que** R⁰ est sélectionné pour chaque occurrence, de manière identique ou différente, parmi le groupe qui est constitué par H, un groupe alkyle en chaîne droite qui comporte de 1 à 10 atome(s) de C ou un groupe alkyle ramifié ou cyclique qui comporte de 3 à 10 atomes de C, dont chacun peut être substitué par un radical ou par plusieurs radicaux R⁴.

13. Procédé pour la préparation d'un composé selon une ou plusieurs des revendications qui précèdent, **caractérisé en ce que** les groupes diarylamino A et N(Ar)² sont introduits au moyen d'une réaction de couplage C-N entre un 1- ou 4- spirobifluorène halogéné et un groupe diarylamine A ou N(Ar)2.

14. Formulation comprenant au moins un composé selon une ou plusieurs des revendications 1 à 12 et au moins un solvant.

15. Utilisation d'un composé selon une ou plusieurs des revendications 1 à 12 dans un dispositif électronique, en particulier dans un dispositif électroluminescent organique.

16. Dispositif électronique comprenant au moins un composé selon une ou plusieurs des revendications 1 à 12, de préférence sélectionné parmi le groupe qui est constitué par les dispositifs électroluminescents organiques, les circuits intégrés organiques, les transistors à effet de champ organiques, les transistors à film mince organiques, les transistors à émission de lumière ou électroluminescents organiques, les cellules solaires organiques, les cellules solaires organiques sensibilisées par colorant, les détecteurs optiques organiques, les photorécepteurs organiques, les dispositifs à extinction de champ organiques, les cellules électrochimiques à émission de lumière ou électroluminescentes, les diodes laser organiques et les dispositifs à émission de plasmons organiques.

17. Dispositif électronique selon la revendication 16, lequel est un dispositif électroluminescent organique, **caractérisé en ce que** le composé selon une ou plusieurs des revendications 1 à 12 est utilisé en tant que matériau de transport de trous dans une couche de transport de trous ou d'injection de trous ou de blocage d'excitons ou de blocage d'électrons ou en tant que matériau de matrice pour les émetteurs fluorescents ou phosphorescents.
